# FASCICULE DE BREVET EUROPEEN

(11) **EP 3 897 212 B1**
(45) Date de publication et mention de la délivrance du brevet: **02.07.2025**
(21) Numéro de dépôt: 19832918.7
(22) Date de dépôt: 19.12.2019
(51) Int. Cl.: A61K 36/064, A61P 1/02, A61K 6/69, A61K 9/00, A23L 33/14

(54) **SOUCHE SACCHAROMYCES CEREVISIAE VAR BOULARDII POUR TRAITER LES MALADIES INFECTIEUSES DE LA CAVITÉ ORALE**
SACCHAROMYCES CEREVISIAE VAR BOULARDII STAMM ZUR BEHANDLUNG VON INFEKTION DER MUNDHÖLE
SACCHAROMYCES CEREVISIAE VAR BOULARDII STRAIN FOR TREATING INFECTIONS OF THE ORAL CAVITY

(30) Priorité: 19.12.2018 FR 1873321
(43) Date de publication de la demande: 27.10.2021
(73) Titulaire: Lesaffre et Compagnie, 75001 Paris (FR)
(72) Inventeur: BALLET, Nathalie, 59700 MARC EN BAROEUL (FR); DECHERF, Amélie, 59910 BONDUES (FR); DEHAY-HARMEL, Elodie, 59840 LOMPRET (FR)
(74) Mandataire: Plasseraud IP
(86) Numéro de dépôt international: PCT/EP2019/086265
(87) Numéro de publication internationale: WO 2020/127699

(56) Documents cités:
- EP-A1- 1 852 122
- WO-A1-2020/127136
- FR-A1- 2 928 652
- FR-A1- 3 005 577
- REZA MOHAMMAD SALEHI ET AL: "Effect of Saccharomyces boulardii Extract on SAP2 Gene Expression and Antifungal Susceptibility of Candida albicans", JUNDISHAPUR JOURNAL OF MICROBIOLOGY, vol. 11, no. 3, 17 February 2018 (2018-02-17), pages 59891, XP055629367, ISSN: 2008-3645, DOI: 10.5812/jjm.59891

## Description

### Domaine de l'Invention

La présente invention concerne le domaine de la santé orale. L'invention porte plus particulièrement sur une souche *Saccharomyces cerevisiae var boulardii* spécifique, utilisée seule ou en combinaison avec une levure *Saccharomyces cerevisiae* sèche inactivée, dans le traitement et/ou la prévention des maladies infectieuses de la cavité buccale que sont les caries dentaires et les maladies parodontales.

### Contexte de l'Invention

Chez l'homme, la cavité orale est un écosystème complexe, ouvert sur l'intérieur, habité par de nombreux microorganismes dont plus de 700 espèces bactériennes détectées (Ghannoum et al., PLoS Pathog., 2010; 6: e1000713; Marsh et al., Periodontol., 2000, 55: 16-35; Paster et al., Periodontol., 2000, 42: 80-87). Pour un même individu, le nombre d'espèces bactériennes résidentes varie de 150 à 250. La plupart de ces espèces sont commensales et nécessaires pour maintenir l'équilibre de cet écosystème. Pourtant, dans certaines situations (apports glucidiques importants, utilisation de tabac, ou changements physiologiques tels que le vieillissement, la puberté ou la grossesse, etc...), une rupture de cet équilibre se produit et peut conduire au développement de maladies infectieuses de la cavité buccale.

Les principales maladies infectieuses de la cavité buccale sont la carie dentaire et les maladies parodontales. La carie dentaire est une maladie polybactérienne qui se manifeste par une déminéralisation des tissus durs de la dent. Cette déminéralisation est due à la production d'acides par les bactéries fermentaires présentes au sein de la plaque dentaire. Les streptocoques du groupe *mutans* ainsi que les Lactobacilles sont considérés comme les principales bactéries cariogènes. Le rapport de l'Organisation Mondiale de la Santé (OMS) sur la santé bucco-dentaire relève la forte prévalence des caries, notamment parmi les plus jeunes. Comparé au reste du monde, le risque de caries durant l'enfance est très supérieur au sein des pays développés (Amérique de Nord, Australie, Europe, Japon), en raison d'une alimentation très riche en sucre dans ces pays.

Les maladies parodontales sont un ensemble de pathologies affectant le parodonte (c'est-à-dire les tissus de soutien de la dent - os et muqueuse gingivale). Ces maladies se divisent en deux grandes catégories: les gingivites et les parodontites. Les gingivites correspondent à toute inflammation limitée au parodonte superficiel. Les parodontites sont des lésions infectieuses avancées du parodonte et font souvent suite aux gingivites. La présence de certaines bactéries et une réponse inflammatoire intense entraînent la destruction du parodonte. Le passage de l'état sain à l'état de maladie parodontale s'accompagne d'une transition progressive à une flore plus riche en bactéries anaérobies et en bactéries à Gram négatif. Ce phénomène porte le nom de dérive anaérobie. Parmi les espèces bactériennes le plus fréquemment impliquées dans les maladies parodontales, on compte: *Fusobacterium nucleatum* et *Porphyromonas gingivalis* dont la co-aggrégation semble améliorer leur survie et leur pathogénicité (Diaz et al., Microbiology, 2002, 148: 467-472; Saito et al., FEMS Immunol. Med. Microbiol., 2008, 54: 349-355; Polak et al., J. Clin. Periodontol., 2009, 36: 406-410).

Le traitement d'une carie dentaire, qui touche seulement l'émail ou la dentine, comprend une obturation au moyen d'un composite ou d'un amalgame d'argent. Lorsque la dent est très endommagée, le dentiste procède à une restauration à l'aide d'une couronne; et si le nerf est atteint, une dévitalisation de la dent doit être réalisée. Une carie non-traitée continue de progresser et les bactéries finissent par attaquer l'os alvéolaire sous la racine, conduisant à un abcès douloureux qu'il est nécessaire de traiter avec des antibiotiques pour ensuite passer au traitement mécanique. Les antibiotiques sont également utilisés dans le traitement des maladies parodontales en combinaison avec le détartrage et le surfaçage radiculaire, le curetage, la chirurgie dentaire ou comme traitement unique afin de réduire les bactéries avant et/ou après ces procédures parodontales courantes.

L'usage intensif des antibiotiques a introduit une pression de sélection aboutissant au développement inquiétant de populations de microorganismes antibiorésistants et à une baisse générale de l'efficacité thérapeutique. Ponctuelles au départ, ces résistances sont devenues massives et préoccupantes. Certaines souches bactériennes sont devenues multi-résistantes, c'est-à-dire résistantes à plusieurs antibiotiques, et d'autres souches sont devenues toto-résistantes, c'est-à-dire résistantes à tous les antibiotiques disponibles. Ce dernier cas est heureusement encore rare, mais le phénomène est en augmentation et place les médecins dans une impasse thérapeutique. Ainsi, dans le contexte actuel d'une antibiorésistance croissante et préoccupante, les scientifiques et les professionnels de santé cherchent à identifier de nouvelles stratégies thérapeutiques, y compris dans le traitement et/ou la prévention des maladies infectieuses de la cavité buccale.

EP 1 852 122 A1 décrit des compositions comprenant *Saccharomyces cerevisiae* var. *boulardii,* seule ou en combination avec *Enterococcus faecium,* pour traiter des maladies de la cavité orale. Les levures agissent en inhibant les bactéries pathogènes, telles que *Prévotella intermedia* et *Porpyhromona gingivalis,* et en rétablissant l'équilibre de la microflore des tissues de la bouche. Les maladies traitées sont par exemple la ginvivite et la parodontose.

### Résumé de l'Invention

Les présents Inventeurs ont trouvé, de manière surprenante, que les souches de levure *Saccharomyces cerevisiae* var. *boulardii,* telle que la souche de levure *Saccharomyces cerevisiae* var. *boulardii* déposée, par le Demandeur, le 21 août 2007 auprès de la CNCM (Collection National de Cultures de Microorganismes, 25 rue du Docteur Roux, 75724 Paris Cedex, 15, France) sous le numéro I-3799, avaient une capacité accrue, comparée à d'autres probiotiques, à inhiber la croissance d'agents parodontopathogènes et cariogènes.

Les présents Inventeurs ont également mis en évidence une synergie entre une souche de levure *Saccharomyces cerevisiae var. boulardii* et une souche de levure *Saccharomyces cerevisiae* sous forme sèche et inactivée, pour inhiber de manière significative la croissance de certains pathogènes parodontaux et cariogènes. Une telle souche de levure *Saccharomyces cerevisiae var. boulardii* est, par exemple, la souche numéro I-3799 déjà décrite plus haut. Une telle souche de levure *Saccharomyces cerevisiae* est, par exemple, la souche *Saccharomyces cerevisiae* déposée, par le Demandeur, le 17 octobre 2007 auprès de la CNCM sous le numéro I-3856.

La présente invention a ainsi pour objet la souche de levure *Saccharomyces cerevisiae var boulardii* déposée, le 21 août 2007, auprès de la CNCM sous le numéro I-3799, ou une combinaison d'une souche de levure *Saccharomyces cerevisiae* var *boulardii* avec la forme sèche inactivée d'une souche de levure *Saccharomyces cerevisiae,* pour utilisation dans la prévention et/ou le traitement d'une maladie infectieuse de la cavité buccale chez un sujet, caractérisée en ce que la maladie infectieuse est une carie dentaire, une gingivite ou une parodontite.

Dans certains modes de réalisation, la levure *Saccharomyces cerevisiae var boulardii* I-3799 ou la souche de levure *Saccharomyces cerevisiae var boulardii* de la combinaison se trouve sous forme sèche vivante.

Dans certains modes de réalisation, la souche de levure *Saccharomyces cerevisiae var boulardii* de la combinaison est la souche déposée, le 21 août 2007, auprès de la CNCM sous le numéro I-3799.

Dans certains modes de réalisation, la souche de levure *Saccharomyces cerevisiae* est la souche déposée, le 17 octobre 2007 auprès de la CNCM sous le numéro I-3856.

Dans certains modes de réalisation, la souche de levure *Saccharomyces cerevisiae var boulardii* I-3799, ou la combinaison d'une souche de levure *Saccharomyces cerevisiae var boulardii* avec la forme sèche inactivée d'une souche de levure *Saccharomyces cerevisiae,* telle que définie plus haut, est comprise dans un complément alimentaire.

Dans certains modes de réalisation, le complément alimentaire est sous forme d'une pastille à sucer, d'un bonbon, d'un chewing-gum, d'une poudre orodispersible ou à diluer dans l'eau sous forme de stick ou de sachet, d'un comprimé à sucer ou à mâcher, d'une gomme à mâcher, d'une gélule, d'un comprimé, de gouttes, ou d'une fiole avec bouchon doseur.

Dans certains modes de réalisation, la souche de levure *Saccharomyces cerevisiae var boulardii* I-3799, ou la combinaison d'une souche de levure *Saccharomyces cerevisiae var boulardii* avec la forme sèche inactivée d'une souche de levure *Saccharomyces cerevisiae,* telle que définie plus haut, est comprise dans une composition para-pharmaceutique ou cosmétique.

Dans certains modes de réalisation, la composition para-pharmaceutique ou cosmétique est sous forme d'un dentifrice, d'un bain de bouche, d'un spray oral, d'une crème ou d'un gel oral, d'une feuille orodispersible, d'une poudre destinée à être saupoudrée directement dans la cavité buccale, d'une poudre orodispersible ou à diluer dans l'eau sous forme de stick ou de sachet, ou d'une fiole avec bouchon doseur.

Dans certains modes de réalisation, la souche de levure *Saccharomyces cerevisiae var boulardii* I-3799 ou la combinaison d'une souche de levure *Saccharomyces cerevisiae var boulardii* avec la forme sèche inactivée d'une souche de levure *Saccharomyces cerevisiae* est comprise dans une composition pharmaceutique, la composition pharmaceutique comprenant une quantité efficace de la souche de levure *Saccharomyces cerevisiae var boulardii,* I-3799 ou de la combinaison, telle que définie plus haut, et au moins un excipient physiologiquement acceptable.

Dans certains modes de réalisation, la composition pharmaceutique est destinée à une administration topique ou à une administration par voie orale.

Dans certains modes de réalisation, la composition pharmaceutique comprend en outre au moins un principe actif pharmaceutique supplémentaire ayant une activité apaisante, anti-irritante, analgésique, antalgique, anti-inflammatoire, cicatrisante, antibiotique, antipyrétique, ou antifongique.

Dans certains modes de réalisation, la souche de levure *Saccharomyces cerevisiae var boulardii* I-3799 ou la combinaison d'une souche de levure *Saccharomyces cerevisiae var boulardii* avec la forme sèche inactivée d'une souche de levure *Saccharomyces cerevisiae* est comprise dans un appareil médical dentaire.

Dans certains modes de réalisation, l'appareil dentaire est un implant dentaire, une couronne dentaire, un bridge dentaire, un onlay dentaire, ou une prothèse dentaire.

Dans certains modes de réalisation, la maladie infectieuse de la cavité buccale est induite par des bactéries parodontopathogènes et/ou des bactéries cariopathogènes.

Dans certains modes de réalisation, les bactéries parodontopathogènes sont choisies parmi *Aggregatibacter actinomycetemcomitans, Fusobacterium nucleatum*)*, Porphyromonas gingivalis, Prevotella intermedia,* et/ou la bactérie cariopathogène est *Streptococcus mutans.*

Dans certains modes de réalisation, la maladie infectieuse de la cavité buccale destinée à être prévenue ou traitée selon la présente invention est un effet secondaire d'un traitement médical, ou est présente ou susceptible de se développer ou de récidiver chez un patient souffrant d'une immunodéficience, ou est présente chez une femme enceinte, une personne âgée, un enfant ou un patient présentant un phénotype hyper-inflammatoire.

Une description plus détaillée de certains modes de réalisation préférés de l'invention est donnée ci-dessous.

### Description Détaillée de l'Invention

Comme mentionné plus haut, la présente invention concerne une souche de levure *Saccharomyces cerevisiae var boulardii* ayant la capacité d'inhiber la croissance d'agents parodontopathogènes et cariogènes et son utilisation, seule ou en combinaison avec une levure *Saccharomyces cerevisiae* sèche et inactivée, dans le traitement et/ou la prévention de maladies infectieuses de la cavité buccale.

### I - Souche de Levure Saccharomyces cerevisiae var boulardii

L'expression "souche de levure" désigne une population relativement homogène de cellules de levure. Une souche de levure est obtenue à partir d'un clone, un clone étant une population de cellules obtenues à partir d'une seule cellule de levure.

Une souche de levure *Saccharomyces cerevisiae var boulardii* qui peut être utilisée dans le contexte de la présente invention est, par exemple, la souche qui a été déposée le 21 août 2007, par le présent Déposant, à la CNCM (Collection Nationale de Cultures de Microorganismes, 25 rue du Docteur Roux, 75724 Paris Cedex 15, France) sous le numéro I-3799.

Cette souche *Saccharomyces cerevisiae var boulardii* a précédemment été décrite, par le présent Déposant lui-même, dans le document WO 2009/103884, où elle est présentée comme étant utile dans la prévention et/ou le traitement de pathologies, désordres ou troubles de l'intestin.

Dans le contexte de la présente invention, une souche de levure *Saccharomyces cerevisiae var boulardii* se trouve sous la forme de cellules de levure obtenues par culture (ou multiplication) de la souche de départ. La culture d'une souche *Saccharomyces cerevisiae var boulardii* peut s'effectuer par n'importe quelle méthode appropriée. Les procédés de culture d'une souche *Saccharomyces cerevisiae var boulardii* sont connus dans l'art, et l'homme du métier sait optimiser les conditions de culture pour chaque souche en fonction de sa nature. Les cellules de levure peuvent par exemple être obtenues par multiplication d'une souche *Saccharomyces cerevisiae var boulardii* dans un milieu de culture comme décrit dans l'ouvrage de référence "Yeast Technology", 2ème Edition, 1991, Reed and Nagodawithana, publié par Van Nostrand Reinhold (ISBN 0-442-31892-8).

Ainsi, par exemple, à l'échelle industrielle, la levure *Saccharomyces cerevisiae var boulardii,* utilisable dans le contexte de la présente invention, peut être obtenue par un procédé comprenant les étapes suivantes :
- culture d'une souche *Saccharomyces cerevisiae var boulardii* (par exemple la souche numéro I-3799) dans un milieu de culture en plusieurs étapes, d'abord en semi-anaérobiose, puis en aérobiose (milieu/atmosphère riche en oxygène) pour obtenir une multiplication des cellules de levure de départ;
- et séparation, par centrifugation, des cellules de levure ainsi produites pour obtenir une crème de levure liquide contenant entre 12% et 25% de matière sèche levure.

La levure *Saccharomyces cerevisiae var boulardii* utilisée dans le contexte de l'invention est sous forme de levure vivante. Le terme "levure vivante", qui est synonyme de "levure active", désigne une population de cellules de levure qui sont métaboliquement actives.

La levure vivante utilisée dans le contexte de la présente invention se présente sous forme d'une levure sèche. Une levure sèche est caractérisée par une teneur faible en eau, et comprend généralement un taux de matière sèche levure supérieur à 90%, de préférence un taux de matière sèche compris entre 92% et 98%, par exemple un taux de matière sèche compris entre 94,5% et 96.5%. Un des avantages d'une levure sèche est sa longue durée de conservation.

Ainsi le procédé de production de la levure *Saccharomyces cerevisiae var boulardii* peut en outre comprendre une étape ultérieure de séchage, pour obtenir une levure sous forme sèche. Cette étape de séchage est suivie ou non d'une opération de broyage. Le séchage peut par exemple être un séchage par lyophilisation, un séchage par lit fluidisé, un séchage en tambour ou un séchage par atomisation. Dans le cas d'un séchage par lyophilisation, un support de type maltodextrine ou lactose peut être utilisé.

### II - Combinaison de la Souche de Levure Saccharomyces cerevisiae var boulardii et de la Souche de Levure Saccharomyces cerevisiae Sèche Inactivée

Comme indiqué plus haut, les présents Inventeurs ont mis en évidence une synergie entre une souche de levure *Saccharomyces cerevisiae var. boulardii* (par exemple la souche de levure *Saccharomyces cerevisiae var. boulardii* numéro I-3799) et une souche de levure *Saccharomyces cerevisiae* sous forme sèche inactivée pour inhiber de manière significative la croissance de bactéries parodontogènes et cariogènes. Les termes "synergie" et "action synergique", qui sont utilisés ici de manière interchangeable, désignent une action coordonnée des deux souches de levure créant un effet plus grand que la somme des effets attendus si elles avaient opéré indépendamment, ou créant un effet que chacune d'entre elles n'aurait pas pu obtenir en agissant isolément.

La levure *Saccharomyces cerevisiae* utilisée en combinaison avec une levure *Saccharomyces cerevisiae var. boulardii* est une levure sèche inactivée. Les termes "levure inactivée" et "levure désactivée", qui sont ici utilisés indifféremment, désignent une levure qui n'est plus vivante, c'est-à-dire une levure dont le métabolisme est irrémédiablement arrêté. Une levure inactivée selon l'invention peut être obtenue par n'importe quelle méthode appropriée. Les techniques appropriées bien connues de l'homme du métier incluent un traitement thermique de la levure, un traitement par atomisation, un séchage en tambour ou une combinaison quelconque de ces traitements. Une levure inactivée se trouve généralement sous forme sèche. Une levure sèche est caractérisée par une teneur faible en eau, et comprend généralement un taux de matière sèche levure supérieur à 90%, de préférence un taux de matière sèche compris entre 92% et 98%, par exemple un taux de matière sèche compris entre 94,5% et 96.5%. Un des avantages d'une levure sèche est sa longue durée de conservation.

La souche de levure *Saccharomyces cerevisiae var. boulardii* utilisée dans la combinaison peut être n'importe quelle souche de levure *Saccharomyces cerevisiae var. boulardii.*

La souche de levure *Saccharomyces cerevisiae* sèche inactivée utilisée dans la combinaison peut être n'importe quelle souche *Saccharomyces cerevisiae.*

Dans certains modes de réalisation particuliers, la souche de levure *Saccharomyces cerevisiae,* utilisée sous forme sèche inactivée dans le contexte de l'invention en combinaison avec une souche *Saccharomyces cerevisiae var boulardii,* est la souche *Saccharomyces cerevisiae* déposée, le 17 octobre 2007, auprès de la CNCM sous le numéro I-3856. Cette souche *Saccharomyces cerevisiae* a précédemment été décrite, par le présent Déposant lui-même, dans le document WO 2009/103884, où elle est présentée comme étant utile dans la prévention et/ou le traitement de pathologies, désordres ou troubles de l'intestin; et dans le document WO 2014/009656, où elle est présentée comme étant efficace pour contrôler la prolifération vaginale de *Candida* et pour prévenir la récurrence des candidoses vaginales ou vulvovaginales.

Dans certains modes de réalisation particuliers, la souche de levure *Saccharomyces cerevisiae var. boulardii,* utilisée dans le contexte de l'invention en combinaison avec la souche de levure *Saccharomyces cerevisiae* sous forme sèche inactivée est la souche de levure *Saccharomyces cerevisiae var boulardii* déposée, le 21 août 2007, auprès de la CNCM sous le numéro I-3799.

Le terme "combinaison", telle qu'utilisé ici, désigne un mélange de *Saccharomyces cerevisiae var boulardii* (par exemple la souche numéro I-3799) et de *Saccharomyces cerevisiae* sous forme sèche inactivée (par exemple la souche numéro I-3856). Le mélange peut être préparé dans n'importe quelles proportions. Ainsi, préférablement, la combinaison comprend un ratio de *Saccharomyces cerevisiae var boulardii I-3799: Saccharomyces cerevisiae* sous forme sèche inactivée allant d'environ 90:10 (w/w = poids/poids) à environ 10:90 (w/w). Ainsi, le ratio de *Saccharomyces cerevisiae var boulardii* I-3799 : *Saccharomyces cerevisiae* sous forme sèche inactivée peut, par exemple, être d'environ 90:10 (w/w), environ 80:20 (w/w), environ 70:30 (w/w), environ 60:40 (w/w), environ 50:50 (w/w), environ 40:60 (w/w), environ 30:70 (w/w), environ 20:80 (w/w) ou environ 10:90 (w/w). Le terme "environ", tel qu'utilisé ici en référence à un nombre, comprend généralement des nombres qui se situent dans une fourchette de 10% dans l'une ou l'autre direction du nombre (supérieur ou inférieur au nombre), sauf dans le cas où ce nombre dépasse 100% d'une valeur possible.

Dans certains modes de réalisation particuliers de l'invention, le ratio de *Saccharomyces cerevisiae var boulardii* I-3799 : *Saccharomyces cerevisiae* I-3856 sous forme sèche inactivée est d'environ 50 : 50 (w/w).

### III - Utilisations de la Souche de Levure Saccharomyces cerevisiae var boulardii et de la Combinaison dans le Traitement et/ou la Prévention des Maladies Infectieuses de la Cavité Buccale

L'invention concerne donc la souche de levure *Saccharomyces cerevisiae var boulardii* numéro I-3799 ou une combinaison d'une souche de levure *Saccharomyces cerevisiae var bourlardii* (par exemple la souche numéro I-3799) avec la forme sèche inactivée d'une souche *Saccharomyces cerevisiae* (par exemple la souche numéro I-3856), pour le traitement et/ou la prévention de maladies infectieuses de la cavité buccale. Il est également décrit ici une méthode de traitement et/ou de prévention d'une maladie infectieuse de la cavité buccale chez un sujet, la méthode comprenant une étape d'administration, au sujet, d'une quantité efficace de la souche *Saccharomyces cerevisiae* numéro I-3799, ou d'une combinaison d'une souche de levure Saccharomyces cerevisiae var bourlardii (par exemple la souche numéro I-3799) avec la forme sèche inactivée d'une souche *Saccharomyces cerevisiae* (par exemple la souche numéro I-3856). L'invention concerne aussi l'utilisation de la souche *Saccharomyces cerevisiae var boulardii* numéro I-3799, ou d'une combinaison d'une souche de levure *Saccharomyces cerevisiae var bourlardii* (par exemple la souche numéro I-3799) avec la forme sèche inactivée d'une souche *Saccharomyces cerevisiae* (par exemple la souche numéro I-3856), pour la fabrication d'un médicament pour le traitement et/ou la prévention de maladies infectieuses de la cavité buccale.

Dans le contexte de la présente invention, on entend par "traitement" une méthode qui a pour but: (1) de retarder ou prévenir le début d'une maladie ou d'une condition clinique; (2) de ralentir ou d'arrêter la progression, l'aggravation ou la détérioration des symptômes de la maladie; (3) d'apporter des améliorations des symptômes de la maladie; et/ou (4) de guérir la maladie. Un traitement peut être administré avant le début de la maladie, pour une action prophylactique (on parle alors de "prévention"), ou il peut être administré après initiation de la maladie, pour une action thérapeutique.

Le terme "sujet" désigne ici un mammifère, et plus particulièrement un être humain, qui a la possibilité d'être victime d'une infection buccale mais qui n'est pas forcément atteint d'une telle infection. Le terme "sujet" ne désigne pas un âge particulier et englobe donc les nouveaux nés, les enfants, les adolescents, les adultes, et les personnes âgées. Le terme "patient" est parfois utilisé ici, à la place de "sujet", lorsque le sujet souffre (c'est-à-dire a été diagnostiqué comme souffrant) d'une maladie infectieuse de la cavité buccale.

Le terme "maladie infectieuse de la cavité buccale" désigne une condition médicale localisée dans la bouche, qui résulte d'une infection par un microorganisme pathogène. Les maladies infectieuses de la cavité buccale incluent les caries dentaires et les maladies parodontales.

Par "carie dentaire" ou "carie", on entend une maladie infectieuse de la dent, qui provoque une lésion de l'émail, de la dentine, et/ou du pulpe/cément. Les bactéries cariogènes, c'est-à-dire les bactéries qui favorisent l'apparition de la carie, incluent : le *Streptococcus mutans* (qui favorise la carie des faces lisses et proximales); le *Streptococcus mutans* et les lactobacilles (qui favorisent la carie des sillons); l'*Actinomycetes viscosus* et l'*Actinomycetes naeslundi* (qui favorisent la carie radiculaire et la colonisation de la dentine et de la racine); et les lactobacilles (qui favorisent la colonisation de la dentine et de la racine). La colonisation des dents commence par les streptocoques, puis les actinomycètes et finalement, les lactobacilles. La prédominance bactérienne varie par rapport à la profondeur de la lésion. Le streptocoque *mutans* est associé aux lésions initiales, alors que les lactobacilles apparaissent plus tard dans une lésion profonde. Les pathogènes suivants sont par exemple aussi reconnus pour être en lien dans la survenue de caries dentaires: *Streptococcus sobrinus* et les *Lactobacilles.*

Par "maladies parodontales", on entend les maladies et conditions pathologiques affectant les tissus de support des dents, c'est-à-dire l'os et la gencive. Ce sont des infections causées par l'accumulation de bactéries pathogènes et de leurs toxines sur le rebord de la gencive au pourtour des dents. Les principales maladies parodontales sont la gingivite et la parodontite. La gingivite est l'inflammation de la gencive autour des dents. Cette condition est réversible lorsqu'elle est traitée. La parodontite, par contre, est une pathologie plus sévère détériorant la gencive et l'os supportant les dents. Les bactéries suivantes sont par exemple aussi reconnues pour être en lien avec la survenue de maladies parodontales: *Porphyromonas gingivalis, Treponema denticola, Tannerella forsythensis, Aggregatibacter actinomycetemcomitans, Actinobacillus actinomycetemcomitans, Actinomycetes odontolyticus, Actinomycetes naeslundii, Fusobacterium Nucleatum, Campylobacter rectus, Peptostreptococcus micros, Prevotella melaninogenica, Prevotella intermedia, Capnocytophaga, Eikenella, Bacteroidetes, Eubacterium saphenum, P. endodontalis, Prevotella denticola, Parvimonas micra, Filifactor alocis,* les espèces *Desulfobulus* et les espèces *Synergistetes.*

D'après la méta-analyse de Guerra *et al.,* 600 espèces bactériennes ont été identifiées dans le microbiome humain oral. Toutefois, 53% des espèces du microbiome oral n'ont pas encore été identifiées et 35% ne sont pas cultivables.

Une méthode de traitement et/ou de prévention d'une maladie infectieuse de la cavité buccale décrite ici comprend l'administration, à un sujet, d'une quantité efficace de la souche *Saccharomyces cerevisiae var boulardii* numéro I-3799, ou d'une combinaison d'une levure *Saccharomyces cerevisiae var boulardii* (par exemple la souche numéro I-3799) avec la forme sèche inactivée de la souche *Saccharomyces cerevisiae* (par exemple la souche numéro I-3856). La quantité efficace, qui peut être administrée en une ou plusieurs doses, peut être déterminée par le médecin ou le dentiste. La quantité exacte à administrer peut varier d'un patient à un autre, en fonction de l'âge, du poids, de la condition générale du patient, de la gravité et/ou de l'étendue de la carie dentaire ou de la maladie parodontale, etc.... La quantité efficace à administrer peut également varier en fonction de l'effet thérapeutique désiré (c'est-à-dire la prévention d'une maladie infectieuse de la cavité buccale ou le traitement d'une maladie infectieuse de la cavité buccale).

Préférablement, l'administration de la souche *Saccharomyces cerevisiae var boulardii* numéro I-3799, ou d'une combinaison d'une levure *Saccharomyces cerevisiae var boulardii* (par exemple la souche numéro I-3799) avec la forme sèche inactivée de la souche *Saccharomyces cerevisiae* (par exemple la souche numéro I-3856), est une administration orale avec un effet localisé à la cavité buccale, cependant dans certains modes de réalisation, la levure (ou la combinaison) peut être avalée (voir plus bas).

Par exemple, la dose de la combinaison d'une levure *Saccharomyces cerevisiae var boulardii* sous forme sèche (par exemple la souche numéro I-3799) et d'une levure *Saccharomyces cerevisiae* sous forme sèche inactivée (par exemple la souche numéro I-3856) peut être comprise entre 1 mg et 10 g, de préférence entre 100 mg et 5 g du mélange.

La concentration recommandée de levure *Saccharomyces* cerevisiae *var. boulardii* vivante peut être comprise entre 1·10⁷ et 1.10¹¹ UFC/g, et de préférence entre 5.10⁸ et 1·10¹⁰ UFC/g.

Une méthode de traitement décrite ici peut être utilisée pour traiter un premier épisode de maladie infectieuse de la cavité buccale, en particulier une gingivite ou une parodontite, ou bien une récidive. Dans un cas comme dans l'autre, le patient peut précédemment avoir été traité par un antibiotique habituel. Alternativement, la levure de *Saccharomyces cerevisiae var boulardii* numéro I-3799, ou la combinaison d'une levure *Saccharomyces cerevisiae var boulardii* (par exemple la souche numéro I-3799) avec une levure de *Saccharomyces cerevisiae* (par exemple la souche numéro I-3856), peut être le premier traitement prescrit au patient.

Une méthode de traitement décrite ici peut également être utilisée pour prévenir une maladie infectieuse de la cavité buccale chez un patient, que la maladie infectieuse de la cavité buccale soit un premier épisode ou une récidive. Cela peut être le cas de patients recevant un traitement médical connu pour engendrer des perturbations du parodonte, comme les immunosuppresseurs, la phénytoine (anti-épileptique), les inhibiteurs calciques comme la nifédipine (qui sont indiqués dans le traitement de troubles cardiaques divers tels que l'angine de poitrine, les arythmies, et l'hypertension artérielle). Cela peut également être le cas de patients souffrant d'une immunovulnérabilité par exemple associée à une maladie comme le syndrome de Sjögren, une infection au VIH, un diabète non contrôlé, certains troubles endocriniens, une malnutrition ou malabsorption comme, par exemple une carence en vitamine B. Cela peut aussi être le cas de patients ayant un débit salivaire réduit (par exemple certaines personnes âgées). Cela peut également être le cas des fumeurs ou des personnes en période de stress. Cela peut aussi être le cas des femmes enceintes dont la prévention des affections bucco-dentaires peut commencer dès le début de la grossesse. Cela peut enfin être le cas de personnes présentant un phénotype hyper-inflammatoire, c'est-à-dire ayant une propension à provoquer une réaction inflammatoire exacerbée en répondant, face à une agression de la même intensité, par une surproduction de cytokines pro-inflammatoires (PGE2, IL-1, TNF), facteurs qui jouent un rôle majeur dans la destruction parodontale.

Dans certains modes de réalisation, un traitement décrit ici est administré seul. En d'autres termes, la levure *Saccharomyces cerevisiae var boulardii* numéro I-3799, ou la combinaison d'une levure *Saccharomyces cerevisiae var boulardii* (par exemple la souche numéro I-3799) avec la forme sèche inactivée d'une levure *Saccharomyces cerevisiae* (par exemple la souche numéro I-3856), est le seul agent à être administré, à l'exception d'éventuels bains de bouche antiseptiques.

Dans d'autres modes de réalisation, un traitement décrit ici est administré en combinaison avec une autre thérapie, par exemple avec l'administration d'un antibiotique ou d'un antiseptique habituel, et/ou avec un procédé mécanique ou chirurgical, comme un détartrage, un surfaçage radiculaire, un curetage, une obturation, une restauration, ou une dévitalisation.

### IV - Compléments Alimentaires, Compositions Cosmétiques et Pharmaceutiques

Comme indiqué plus haut, la souche *Saccharomyces cerevisiae var boulardii* numéro I-3799, ou la combinaison d'une levure *Saccharomyces cerevisiae var boulardii* (par exemple la souche numéro I-3799) avec la forme sèche inactivée d'une souche *Saccharomyces cerevisiae* (par exemple la souche numéro I-3856), peut être administrée comme telle ou sous la forme d'une préparation ou d'une composition.

Ainsi, dans certains modes de réalisation, la souche *Saccharomyces cerevisiae var boulardii* numéro I-3799, ou la combinaison d'une levure *Saccharomyces cerevisiae var boulardii* (par exemple la souche numéro I-3799) avec la forme sèche inactivée d'une souche *Saccharomyces cerevisiae (*par exemple la souche numéro I-3856), est présente dans un complément alimentaire sous forme d'une pastille à sucer, d'un bonbon, d'un chewing gum, d'une poudre orodispersible ou à diluer dans l'eau sous forme de stick ou de sachet, d'un comprimé à sucer ou à mâcher, d'une gomme à mâcher, d'une gélule, d'un comprimé, de gouttes, ou d'une fiole avec bouchon doseur, etc....

Dans d'autres modes de réalisation, la souche *Saccharomyces cerevisiae var boulardii* numéro I-3799, ou la combinaison d'une levure *Saccharomyces cerevisiae var boulardii* (par exemple la souche numéro I-3799) avec la forme sèche inactivée d'une souche *Saccharomyces cerevisiae (*par exemple la souche numéro I-3856), est présente dans une composition para-pharmaceutique (ou cosmétique) sous forme d'un dentifrice, d'un bain de bouche, d'un spray oral, d'une crème ou d'un gel oral, d'une feuille orodispersible, ou encore d'une poudre qui peut être saupoudrée directement dans la cavité buccale, d'une poudre orodispersible ou à diluer dans l'eau sous forme de stick ou de sachet d'une fiole avec bouchon doseur etc...

Dans encore d'autres modes de réalisation, la souche *Saccharomyces cerevisiae var boulardii* numéro I-3799, ou la combinaison d'une levure *Saccharomyces cerevisiae var boulardii* (par exemple la souche numéro I-3799) avec la forme sèche inactivée d'une souche *Saccharomyces cerevisiae (*par exemple la souche numéro I-3856), est présente dans une composition pharmaceutique avec au moins un excipient physiologiquement acceptable. Ainsi, plus spécifiquement, une composition pharmaceutique selon l'invention comprend une quantité efficace de la souche de levure *Saccharomyces cerevisiae var boulardii* numéro I-3799, ou de la combinaison d'une levure *Saccharomyces cerevisiae var boulardii* (par exemple la souche numéro I-3799) avec la forme sèche inactivée d'une souche *Saccharomyces cerevisiae (*par exemple la souche numéro I-3856), et au moins un excipient physiologiquement acceptable. Une composition pharmaceutique selon l'invention peut être classée en tant que préparation pharmaceutique disponible sur ordonnance ou en vente libre.

Dans le contexte de la présente invention, on entend par "excipient physiologiquement acceptable" tout milieu ou additif qui n'interfère pas avec l'efficacité de l'activité biologique du principe actif (ici la souche *Saccharomyces cerevisiae var boulardii* numéro I-3799 ou la combinaison), et qui n'est pas excessivement toxique pour le patient ou sujet, aux concentrations auxquelles il est administré. Un excipient physiologiquement acceptable peut être un excipient approprié à l'administration aux mammifères, en particulier aux humains.

Les compositions pharmaceutiques selon la présente invention peuvent être administrées en utilisant toute combinaison de dosage et de voie d'administration efficace pour obtenir l'effet thérapeutique/prophylactique désiré. Comme déjà indiqué plus haut, la quantité exacte à administrer peut varier d'un patient à un autre, en fonction de l'âge, du poids, de la condition générale du patient, de la gravité et de l'étendue de l'infection buccale et de la nature de la maladie bucco-dentaire associée (carie dentaire, gingivite, parodontite), etc... La voie d'administration (topique ou systémique) peut être choisie en fonction de la gravité et de l'étendue de bactérienne et/ou en fonction de l'âge et/ou de la santé du patient.

A titre d'exemple, l'invention a pour objet une composition pharmaceutique telle que définie ci-dessus pour une utilisation d'une combinaison de la levure *Saccharomyces cerevisiae var boulardii* (par exemple la souche numéro I-3799) et de la forme sèche inactivée d'une levure *Saccharomyces cerevisiae* (par exemple la souche numéro I-3856) dans une quantité comprise entre 1 mg et 10 g, de préférence entre 100 mg et 5 g, cette quantité pouvant être administrée en une ou plusieurs fois par jour.

La concentration recommandée de levure *Saccharomyces* cerevisiae *var. boulardii* peut être comprise entre 1·10⁷ et 1.10¹¹ UFC/g, et de préférence entre 5.10⁸ et 1·10¹⁰ UFC/g.

La formulation d'une composition pharmaceutique selon la présente invention peut varier en fonction de la voie d'administration et du dosage pour lesquels la composition est destinée à être utilisée. Après formulation avec au moins un excipient physiologiquement acceptable, une composition pharmaceutique de l'invention peut être sous toute forme appropriée pour l'administration à un être humain, par exemple sous la forme de tablettes, comprimés, dragées, capsules, perles, sirops, émulsions, onguents, pâtes, gels, poudres, sachets, solutions injectables, etc. L'homme de l'art sait sélectionner les véhicules et excipients les plus appropriés à la préparation d'un type donné de formulation. Une composition selon l'invention peut en outre comprendre des additifs comme des conservateurs, des édulcorants, des arômes, des agents viscosants, des colorants, des agents humectants, des agents de désintégration, des accélérateurs d'absorption, des agents lubrifiants, etc.

Dans certains modes de réalisation, une composition pharmaceutique selon l'invention ne contient qu'un seul agent actif: la levure *Saccharomyces cerevisiae var boulardii* numéro I-3799 ou la combinaison d'une levure *Saccharomyces cerevisiae var boulardii* (par exemple la souche numéro I-3799) avec la forme sèche inactivée d'une levure *Saccharomyces cerevisiae* (par exemple la souche numéro I-3856). Une telle composition pharmaceutique ne contient pas, en particulier, un autre microorganisme vivant ou une combinaison de microorganismes vivants.

Dans d'autres modes de réalisation, une composition pharmaceutique selon l'invention contient, en outre, au moins un principe actif pharmaceutique supplémentaire (c'est-à-dire, en plus de la levure *Saccharomyces cerevisiae var boulardii* I-3799 ou des levures de la combinaison). On entend par "principe actif pharmaceutique", tout composé ou substance dont l'administration a un effet thérapeutique ou dont l'administration a un effet bénéfique à la santé ou à la condition générale d'un patient ou d'un sujet auquel il est administré.

Ainsi, un principe actif pharmaceutique peut être actif contre une infection bactérienne de la cavité buccale que l'on veut prévenir ou traiter par administration de la composition pharmaceutique; ou peut être actif contre une condition ou un symptôme associé à la maladie infectieuse de la cavité buccale (par exemple des douleurs ou de la fièvre ou une halitose); ou encore peut accroître la disponibilité et/ou l'activité du ou des principes actifs de la composition pharmaceutique.

Des exemples de principes actifs pharmaceutiques qui peuvent être présents dans une composition de la présente invention incluent, sans limitation, les principes actifs ayant une activité apaisante, anti-irritante, analgésique, antalgique, anti-inflammatoire, cicatrisante, antibiotique, antipyrétique, ou encore antifongique (sans effet sur la levure *Saccharomyces cerevisiae var boulardii* numéro I-3799 ou sur la combinaison d'une levure *Saccharomyces cerevisiae var boulardii* (par exemple la souche numéro I-3799) avec une levure *Saccharomyces cerevisiae* sous forme sèche inactivée (par exemple la souche numéro I-3856)), etc....

Dans encore d'autres modes de réalisation, la souche *Saccharomyces cerevisiae var boulardii* numéro I-3799, ou la combinaison d'une *souche Saccharomyces cerevisiae var boulardii* (par exemple la souche numéro I-3799) avec la forme sèche inactivée d'une souche *Saccharomyces cerevisiae (*par exemple la souche numéro I-3856), est présente sur/dans un appareil médical dentaire: par exemple un implant dentaire, une couronne dentaire, un bridge dentaire, un onlay dentaire, une prothèse dentaire, etc.

A moins qu'ils ne soient définis d'une autre manière, tous les termes techniques et scientifiques utilisés dans la Description ont la même signification que celle couramment comprise par un spécialiste ordinaire du domaine auquel appartient cette invention.

### Exemples

Les exemples suivants décrivent certains modes de réalisation de la présente invention. Cependant, il est entendu que les exemples et les figures ne sont présentés qu'à titre illustratif et ne limitent en aucun cas la portée de l'invention.

### Légende des Figures

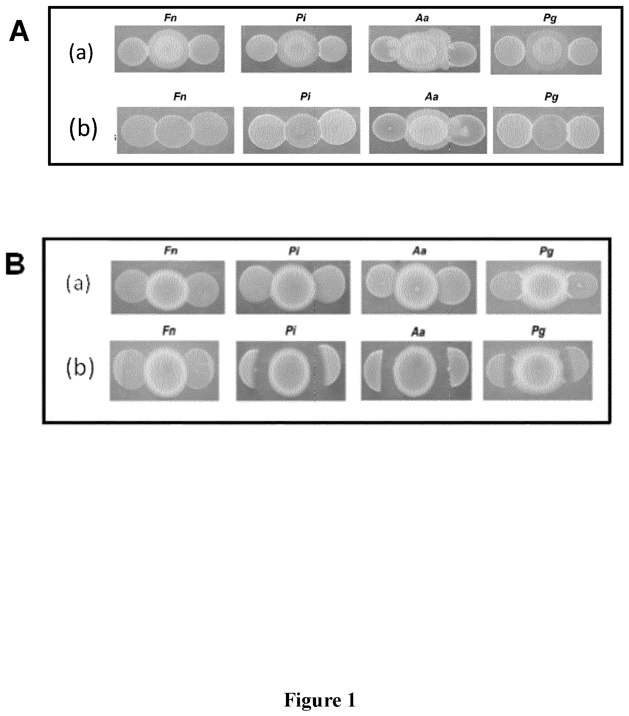

**Figure 1****:** Résultats de halos obtenus **(A)** avec la souche *Saccharomyces cerevisiae* numéro I-3856, en présence des espèces pathogènes : *Fusobacterium nucleatum* (Fn), *Prevotella intermedia* (Pi), *Aggregatibacter actinomycetemcomitans* (Aa), *Porphyromonas gingivalis* (Pg) **(B)** avec la souche *Saccharomyces cerevisiae var boulardii* numéro I-3799, en présence des espèces pathogènes : *Fusobacterium nucleatum* (Fn), *Prevotella intermedia* (Pi), *Aggregatibacter actinomycetemcomitans* (Aa), et *Porphyromonas gingivalis* (Pg) ; **(a)** en aérobie ou **(b)** en anaérobie.

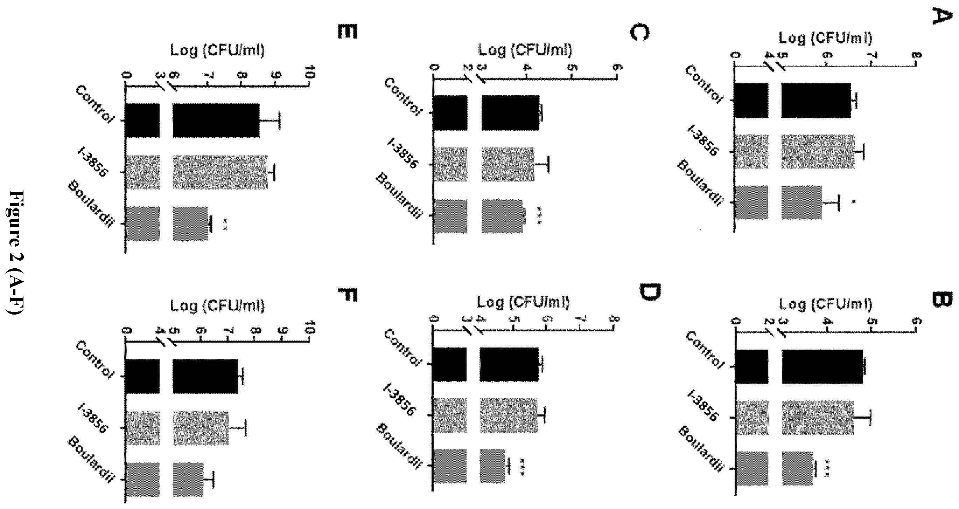

**Figure 2****:** Effets de la souche *Saccharomyces cerevisiae* numéro I-3856 (I-3856); de la souche *Saccharomyces cerevisiae var boulardii* numéro I-3799 (Boulardii); et d'un contrôle (Control) sur différentes espèces bactériennes d'un biofilm de 14-espèces, dont 4 parodontopathogènes **(A** à **D),** et 2 cariopathogènes (**E** et **F**): **(A)** *Aggregatibacter actinomycetemcomitans* (Aa), **(B)** *Fusobacterium nucleatum* (Fn), **(C)** *Porphyromonas gingivalis* (Pg), **(D)** *Prevotella intermedia* (Pi), **(E)** *Streptococcus mutans* (Sm), **(F)** *Streptococcus sobrinus.* Les valeurs représentent la moyenne ± SE à partir de triplicatas (N=3). *p<0,05 vs. Contrôle ; **p<0,01 vs. Contrôle; ***p<0,001 vs. Contrôle; **** p<0,0001 vs. Contrôle.

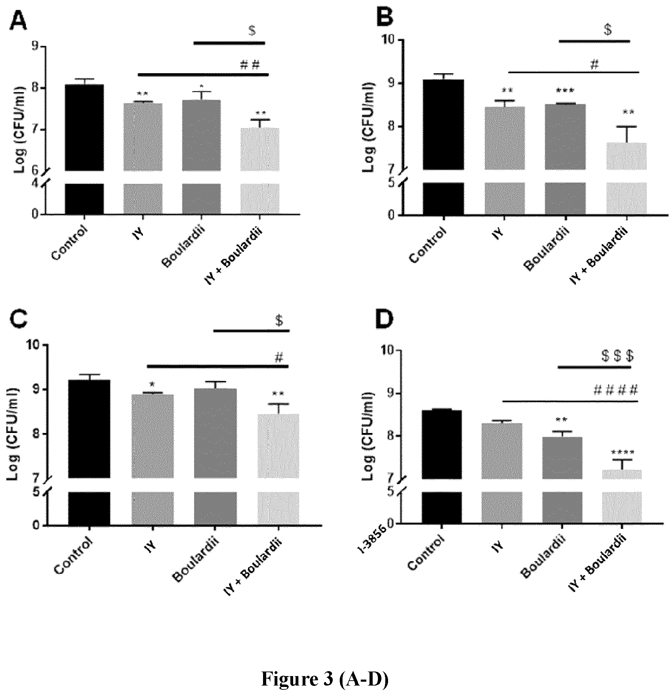

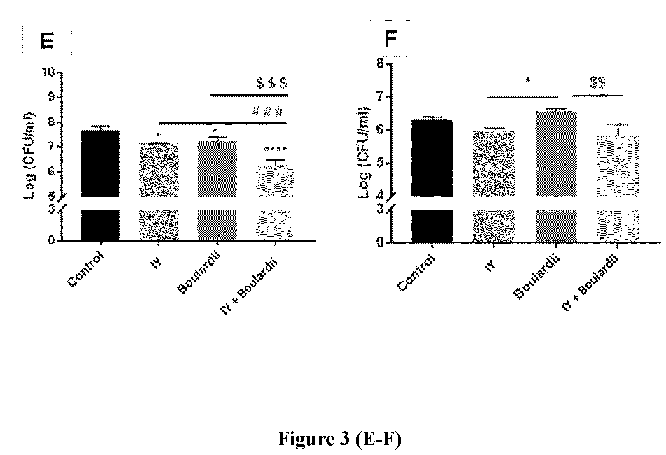

**Figure 3****:** Effets de la forme sèche inactivée de la souche *Saccharomyces cerevisiae* numéro I-3856 (IY)); de la souche *Saccharomyces cerevisiae var boulardii* numéro I-3799 (Boulardii); de la combinaison de la forme sèche inactivée de la souche *Saccharomyces cerevisiae* numéro I-3856 et de la souche *Saccharomyces cerevisiae var boulardii* numéro I-3799 (IY+Boulardii) ; et d'un contrôle (Control) sur différentes espèces bactériennes d'un biofilm de 14-espèces, dont 4 parodontopathogènes (**A** à **D**), et 2 cariopathogènes (**E** et **F**): **(A)** *Aggregatibacter actinomycetemcomitans* (Aa), **(B)** *Fusobacterium nucleatum* (Fn), **(C)** *Porphyromonas gingivalis* (Pg), **(D)** *Prevotella intermedia* (Pi), **(E)** *Streptococcus mutans* (Sm), **(F)** *Streptococcus sobrinus.* Les valeurs représentent la moyenne ± SE à partir de triplicatas (N=3). *p<0,05 vs. Contrôle; **p<0,01 vs. Contrôle; ***p<0,001 vs. Contrôle; **** p<0,0001 vs. Contrôle. # p<0,05 vs. Nutri. $ p<0,05 vs. Boulardii.

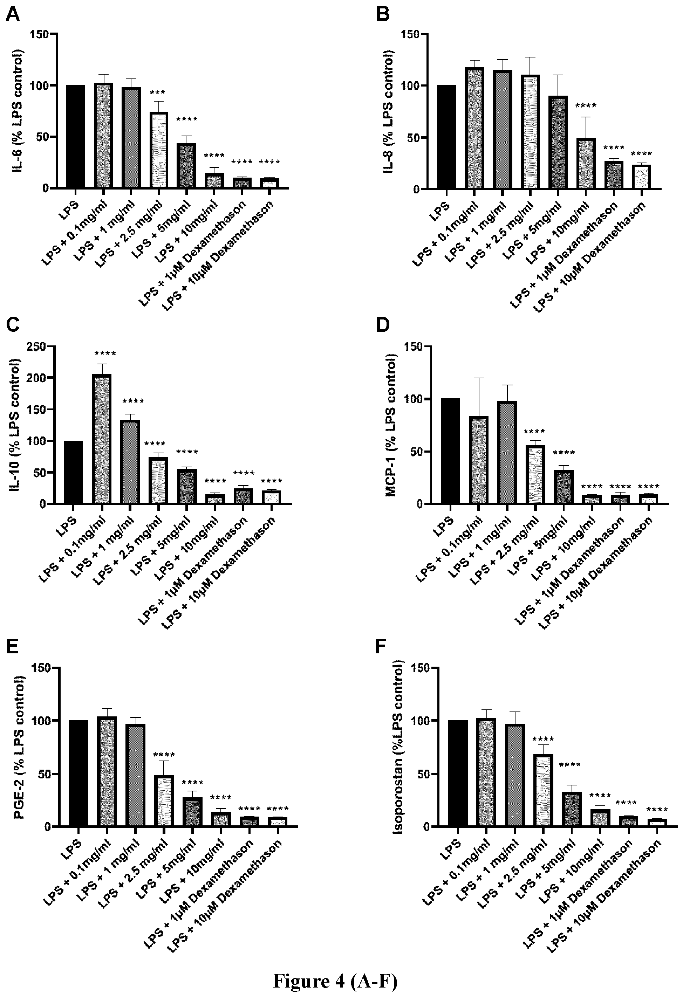

**Figure 4****:** Courbes de dose-effets de la levure *Saccharomyces cerevisiae* var. *boulardii* CNCM I-3799 sur la sécrétion des différents paramètres inflammatoires suivants : (**A**) IL-6, (**B**) IL-8, (**C**) IL-10, (**D**) MCP-1, (**E**) PGE-2, et (**F**) isoprostane, par des monocytes primaires humaines après un challenge LPS. Les valeurs sont exprimées en moyenne ± SE à partir d'expériences dupliquées effectuées pour trois donneurs (n=6). *** p<0,001 vs. Contrôle, **** p<0,0001 vs. Contrôle.

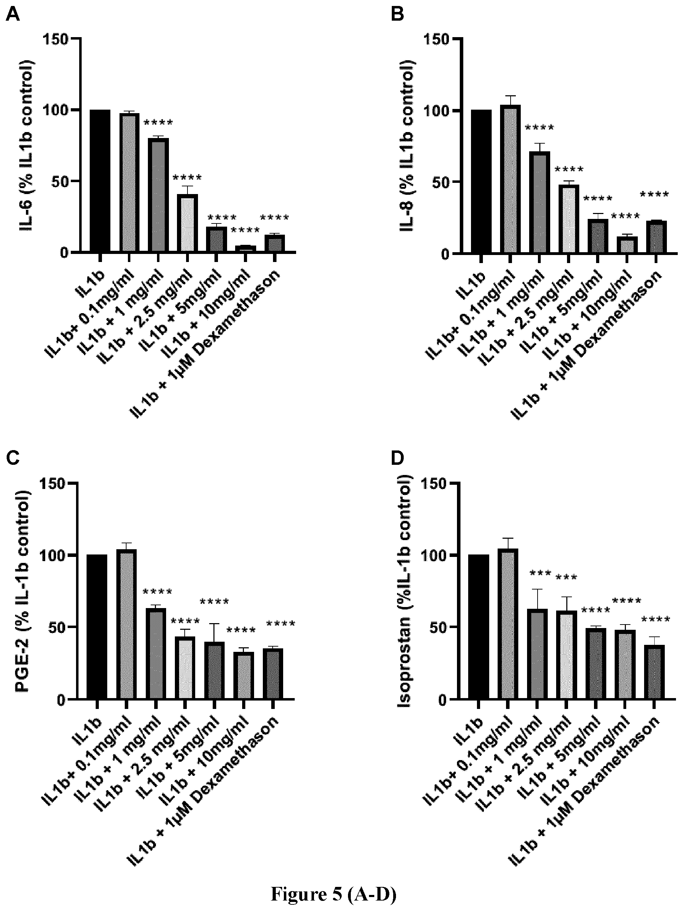

**Figure 5****:** Courbes de dose-effets de la levure *Saccharomyces cerevisiae* var. *boulardii* CNCM I-3799 sur la sécrétion des différents paramètres inflammatoires suivants : (**A**) IL-6, (**B**) IL-8, (**C**) PGE-2, et (**D**) isoprostane, par des fibroblastes gingivaux primaires humains. Les valeurs sont exprimées en moyenne ± SE pour trois expériences (n=3). *** p<0,001 vs. Contrôle, **** p<0,0001 vs. Contrôle.

### Effets de Différents Probiotiques sur des Bactéries Parodontopathogènes et des Bactéries Cariogènes

### A. Probiotiques Testées et Bactéries Parodontopathogènes ou Cariogènes

***Produits de Levures.*** Dans les études décrites ci-dessous, 2 levures sèches vivantes, et une levure sèche inactivée ont été évaluées.

Les deux levures vivantes sont:
- La souche *Saccharomyces cerevisiae* numéro CNCM I-3856 (déposée, par le Déposant, à la CNCM le 17 octobre 2007), et
- La souche *Saccharomyces cerevisiae var boulardii* numéro CNCM I-3799 (déjà décrite plus haut).

La levure sèche inactivée est:
- La forme sèche inactivée de la souche *Saccharomyces cerevisiae* numéro I-3856 (déjà décrite plus haut).

### Composition d'un Biofilm complexe à 14 espèces:

*Bactéries Parodontopathogènes et Bactéries Cariogènes.* Parmi les bactéries parodontopathogènes utilisées, on trouve :
- *Prevotella intermedia* (anciennement connu sous le nom de *Bacteroides intermedius*) qui est une bactérie pathogène anaérobie obligatoire, à Gram négatif, impliquée dans les infections parondontales, y compris la gingivite et la parodontite, et souvent retrouvée dans la gingivite ulcéreuse nécrosante aiguë;
- *Porphyromonas gingivalis,* qui est une bactérie pathogène anérobie, non-motile, à Gram négatif, sous forme de bâtonnet, que l'on retrouve dans la cavité buccale, où elle est impliquée dans certaines formes de maladies parodontales;
- *Fusobacterium nucleatum,* qui est une bactérie anérobie, invasive, adhérente et inflammatoire, et étrangère à la cavité buccale de l'homme, et qui joue un rôle dans la maladie parodontale en étant un élément clé de la plaque parodontale en raison de son abondance et de sa capacité à s'associer avec d'autres espèces dans la cavité buccale; et
- *Aggregatibacter actinomycetemcomitans,* qui est une petite bactérie à Gram négatif, à croissante lente, aéro-anaérobie facultative, appartenant à la flore buccale physiologique de l'homme, et jouant un rôle dans la maladie parodontale.

Parmi les bactéries cariogènes utilisées, on trouve:
- *Streptococcus mutans,* qui est une bactérie cocci de type Gram positif, faisant partie de la flore commensale de la cavité buccale où elle est responsable de la carie dentaire en combinaison avec le sucre alimentaire qu'elle transforme en acide lactique ; et
- *Streptococcus sobrinus,* qui est une bactérie à Gram négatif, non motile et anaérobie, présente en grandes quantités dans la plaque dentaire des patients ayant des caries.

*Bactéries Commensales de la Cavité Buccale.* Parmi les bactéries commensales de la cavité buccale utilisées dans la présente étude, on trouve : *Streptococcus sanguinis*; *Streptococcus gordonii*; *Streptococcus salivarius, Streptococcus mitis*; *Streptococcus oralis; Actinomyces viscosus*; *Actinomyces naeslundii*; et *Veillonella parvula.*

### B. Activité Antibactérienne des Levures contre 6 Espèces pathogènes

***Protocole.*** La technique du halo a été utilisée pour détecter et quantifier le taux d'inhibition des bactéries commensales et pathogéniques sur des plaques d'agar. Cette technique est un test compétitif, qui consiste à inoculer deux spots, proches l'un de l'autre et contenant chacun une espèce bactérienne différente, sur une plaque de gélose (agar). Ensuite, la capacité de l'une à inhiber la croissance de l'autre peut être évaluée.

Les plaques d'agar ont été ensemencées avec la culture de l'une des 2 levures (vivantes) ajustée à une densité optique à 600 nm (OD₆₀₀) de 0,5, ce qui correspond à une concentration d'environ 10⁸ CFU/ml - les plaques d'agar BHI-2 ne contenant pas d'hémine, de ménadion ou de sang. Chaque spot contenait 7 µL de la culture de l'une des 2 levures vivantes de concentration 10⁸ CFU/ml.

Après 24 heures d'une incubation aérobie ou anaérobie, les plaques d'agar ont de nouveau été ensemencées pendant 24h avec 7 µl d'une culture d'une espèce pathogène (*Fusobacterium nucleatum* (Fn), *Prevotella intermedia* (Pi), *Aggregatibacter actinomycetemcomitans* (Aa), et *Porphyromonas gingivalis* (Pg), tout près du spot contenant l'espèce levure.

Au total, les plaques d'agar ont été incubées pendant 48 heures en anaérobie ou en aérobie. Après l'incubation de 48 heures, la surface d'inhibition a été inspectée et calibrée (avec une règle) (Tableau 1), et une photographie standardisée (distance entre la plaque d'agar et l'appareil photo) a été prise de chacune des plaques d'agar (Figure 1).

Les pathogènes testés avec les 2 levures vivantes sont les suivants : *Fusobacterium nucleatum* (Fn), *Prevotella intermedia* (Pi), *Aggregatibacter actinomycetemcomitans* (Aa), *et Porphyromonas gingivalis* (Pg),

***Résultats.*** Les résultats obtenus sont présentés sur la Figure 1 et résumés dans le Tableau 1.

**Tableau 1. Taux d'inhibition (moyenne ± déviation standard, N=3) induite par l'espèce levure Saccharomyces cerevisiae var. boulardii I-3799 sur la croissance de certaines bactéries pathogènes orales.**

| **Compétition Bactérienne** | **distance d'inhibition moyenne ± SD* (mm)** |
|---|---|
| *S. boulardii* (24 h anaérobie) + *P. intermedia* (24 h anaérobie) | 6,19 ± 0,09 |
| *S. boulardii* (24 h anaérobie) + *P. gingivalis* (24 h anaérobie) | 3,11 ± 0,14 |
| *S. boulardii* (24 h anaérobie) + *A. actinomycetemcomitans* (24 h aérobie) | 4,48 ± 0,14 |
| *S. boulardii* (24 h aérobie) + *A. actinomycetemcomitans* (24 h aérobie) | 0,52 ± 0,07 |

| | |
|---|---|
| *SD = déviation standard | |

Le Tableau 1 renseigne les distances d'inhibition mesurées pour les cas où la croissance de certains pathogènes oraux a été inhibée de manière significative.

Les résultats obtenus montrent que la souche *Saccharomyces cerevisiae var boulardii* induit une inhibition significative de la croissance des espèces pathogènes *Prevotella intermedia, Aggregatibacter actinomycetemcomitans,* et *Porphyromonas gingivalis* dans des conditions d'anaérobie.

De plus, la croissance d*'Aggregatibacter actinomycetemcomitans* a également été inhibée par la souche *Saccharomyces cerevisiae var boulardii* dans des conditions d'aérobie.

Le Tableau 1. montre que le plus grand ordre de grandeur d'inhibition a été observé sur la croissance de *Prevotella intermedia* en conditions d'anaérobie par comparaison aux autres bactéries et pathogènes.

On observe également sur la Figure 1 que la souche *Saccharomyces cerevisiae* CNCM I-3856 n'a montré aucune inhibition significative des espèces pathogènes.

***Conclusion.*** Seule la souche *Saccharomyces cerevisiae var boulardii* a inhibé la croissance des pathogènes oraux sur les plaques d'agar.

### C. Evaluation de l'Effet des Produits de Levures sur la Composition d'un Biofilm complexe comprenant 14 Espèces.

### 1) Effets des levures vivantes Etudiées

***Protocole Bioréacteur.*** Une communauté multi-espèces a été établie dans un réacteur BIOSTAT B TWIN^{®} (Sartorius, Allemagne). Un milieu contenant 750 ml de BHI-2 (Brain Heart Infusion (BHI) supplémenté avec 2,5 g/l de mucine, 1,0 g/l d'extrait de levure, 0,1 g/l de cystéine, 2,0 g/l de bicarbonate de sodium et 0,25% (v/v) d'acide glutamique) a été ajouté au bioréacteur ainsi que 5,0 mg/ml d'hémine, 1,0 mg/ml de ménadione et 200 µl/l d'Antifoam Y-30 (Sigma, St. Louis, Etats-Unis). Le mélange a été pré-réduit pendant 24 heures à 37°C en faisant buller 100% d'azote (N₂) et 5% de gaz carbonique (CO₂) dans le mélange tout en remuant continuellement à 300 tours/minutes, à un pH fixé à 6,7 ± 0,1. Après 24 heures, les cultures de *Streptococcus mutans (E), Streptococcus sobrinus (F), Prevotella intermedia (D), Porphyromonas gingivalis (C), Fusobacterium nucleatum (B),* et *Aggregatibacter actinomycetemcomitans (A)* ont été ajoutées à une densité optique de 1,4 et ajoutées au mélange du bioréacteur. Le milieu n'a pas été remplacé pendant les 48 premières heures, puis il a ensuite été remplacé à une vitesse de 200 ml/24 heures.

***Protocole Cultures.*** Les cultures de la souche *Saccharomyces cerevisiae* numéro I-3856 (I-3856) et de la souche *Saccharomyces cerevisiae var boulardii* numéro I-3799 (*Boulardii*) (réalisées pendant la nuit) ont été ajustées à une densité optique à 600 nm (OD₆₀₀) de 0,5 (~1x10⁸ CFU/ml) dans du BHI-2 (voir composition ci-dessus). Des aliquotes de 1800 µl de ces cultures ajustées ont été inoculées sur des plaques de 24 puits contenant des disques d'hydroxyapatite au fond de chaque puits. 200 µl du mélange du biofilm à 14 espèces issu du bioréacteur a été ajouté à chaque puits. Les contrôles contenaient 200 µl des échantillons des 14 espèces et 1800 µl de BHI-2. Les plaques de 24 puits ont été incubées dans des conditions de micro-aérobie (6% d'oxygène) pendant 24 heures. Ensuite, les surnageants ont été éliminés et les biofilms attachés aux disques d'hydroxyapatite ont été lavés avec du PBS (phosphate buffered saline). Les biofilms ont été détachés avec 1500 µl de 0,05% de Trypsine-EDTA pendant 45 minutes à 37°C et 230 tours par minutes, puis transférés dans des tubes Eppendorf, et centrifugés (6010 x g, pendant 5 minutes). Après élimination de la trypsine, le culot de biofilm a été re-suspendu dans 500 µl de PBS, et une extraction d'ADN a été réalisée selon les instructions de Qiagen et analysée par qPCR.

***Résultats Obtenus sur les Levures Vivantes.*** Les résultats obtenus sont présentés sur la Figure 2 (A- F).

On observe que la souche *Saccharomyces cerevisiae var boulardii* est capable d'inhiber significativement les parodontopathogènes suivants *Aggregatibacter actinomycetemcomitans (A), Fusobacterium nucleatum (B), Porphyromonas gingivalis* (C) *et, Prevotella intermedia* (D) ainsi que le cariopathogène *Streptococcus mutans* (E)*.*

***Conclusion.*** Les expériences de biofilms ont confirmé, dans des communautés multi-espèces, l'activité inhibitrice de la souche *Saccharomyces cerevisiae var boulardii* contre les pathogènes oraux, qui avait été mise en évidence ci-dessus dans des expériences sur plaques d'agar.

### 2) Effets de la Souche Saccharomyces cerevisiae var boulardii I-3799 (levure boulardii), de la Forme Sèche Inactivée de la Souche Saccharomyces cerevisiae I-3856 (levure IY), et de leur Combinaison

Les produits de levures (boulardii et IY) ont chacun été dissous dans 100 ml de PBS à 37°C et passés au vortex pendant 3 minutes. 10 ml de ces solutions ont été centrifugés à 6010 x g pendant 10 minutes. Les surnageants ont été éliminés et le culot de la levure « Boulardii » a été suspendu dans du BHI-2 en ajustant la concentration à une moyenne de 5x10⁸ CFU/ml. Le culot de la levure « IY » a été suspendu dans une solution contenant un échantillon du mélange du bioréacteur dilué à 1/5.
- Dans un premier puits, ont été placés: 1 ml de la solution de la levure « Boulardii » suspendue dans du BHI-2 et 1 ml du mélange du bioréacteur dilué à 1/5 sans « IY » (condition « Boulardii » seule)
- Dans un deuxième puits, ont été placés: 1 ml de la solution de la levure « IY » et du mélange du bioréacteur dilué à 1/5 et 1 ml de BHI-2 sans « boulardii » (condition IY seule).
- Dans un troisième puits, ont été placés : 1 ml de la solution de la levure « Boulardii » suspendue dans du BHI-2 et 1 ml de la solution de la levure « IY » et du mélange du bioréacteur dilué à 1/5 (condition combinaison Boulardii + IY).
- Dans un quatrième puits, ont été placés : 1 ml du mélange du bioréacteur dilué à 1/5 et 1 ml de BHI-2. Ce puits constitue un puits contrôle.

Les plaques de 24 puits ont été incubées dans des conditions de micro-aérobie (6% d'oxygène) pendant 24 heures. Ensuite, les surnageants ont été éliminés et les biofilms attachés aux disques d'hydroxyapatite ont été lavés avec du PBS. Les biofilms ont été détachés avec 1500 µL de 0,05% de Trypsine-EDTA pendant 45 minutes à 37°C et 230 tours par minutes, puis transférés dans des tubes Eppendorf, et centrifugés (6010 x g, pendant 5 minutes). Après élimination de la trypsine, le culot de biofilm a été re-suspendu dans 500 µl de PBS, et une extraction d'ADN a été réalisée selon les instructions de Qiagen et analysée par qPCR.

***Résultats.*** Les résultats obtenus sont présentés sur la Figure 3 et dans le tableau 2.

**Tableau 2. Ecart entre les valeurs du contrôle et les valeurs des espèces pathogènes du biofilm (ci-après δ) en présence de la souche Saccharomyces cerevisiae var boulardii I-3799 (Boulardii), de la forme sèche inactivée de la souche Saccharomyces cerevisiae 1-3856 (IY), et de leur combinaison.**

| Espèces pathogènes | δ IY | δ Boulardii | Somme δ IY + δ Boulardii | δ Combinaison IY + Boulardii |
|---|---|---|---|---|
| Aa | 0,46 | 0,38 | 0,84 | 1,1 |
| Fn | 0,65 | 0,59 | 1,24 | 1,47 |
| Pg | 0,32 | 0,2 | 0,52 | 0,77 |
| Pi | 0,3 | 0,6 | 0,9 | 1,38 |
| Sm | 0,51 | 0,43 | 0,94 | 1,4 |

| | | | | |
|---|---|---|---|---|
| NB: Les valeurs présentées dans le tableau 2 ne sont pas des valeurs absolues. Elles correspondent à la différence entre les valeurs de contrôle et les valeurs des pathogènes. | | | | |

Ces résultats montrent que chacune des deux souches individuellement (*Saccharomyces cerevisiae var boulardii* (Boulardii) et *Saccharomyces cerevisiae* inactivée (IY) et leur combinaison sont capables d'inhiber de manière significative la croissance de deux pathogènes parodontaux *Aggregatibacter actinomycetemcomitans* ou Aa (A) et *Fusobacterium nucleatum* ou Fn (B) et de la bactérie cariogénique *Streptococcus mutans* ou Sm (E). De plus, le tableau 2 montre que le δ de la combinaison IY + Boulardii est supérieur à la somme des δ IY et δ Boulardii pris individuellement.

Selon la figure 3 (C), la croissance de *Porphyromonas gingivalis* ou Pg (C) a été inhibée par la levure *Saccharomyces cerevisiae* inactivée (IY) ainsi que par la combinaison mais ne l'a pas été de manière significative par la souche *Saccharomyces cerevisiae var boulardii* (Boulardii). De plus, on peut observer une différence significative de l'inhibition entre la levure inactivée (IY) et la combinaison Boulardii + IY démontrant ainsi une action synergique de la combinaison sur l'inhibition de la croissance de *Porphyromonas gingivalis* (C), confirmée par le tableau 2.

Selon la figure 3 (D), la croissance de *Prevotella intermedia* ou Pi (D) a été inhibée par la souche *Saccharomyces cerevisiae var boulardii* ainsi que par la combinaison mais ne l'a pas été de manière significative par la levure inactivée (IY). De plus, on peut observer une différence significative de l'inhibition entre la souche Boulardii et la combinaison Boulardii + IY démontrant ainsi une action synergique de la combinaison sur l'inhibition de la croissance de *Prevotella intermedia* (D), confirmée par le tableau 2.

***Conclusion.*** Ainsi, il a été démontré que la combinaison de la souche *Saccharomyces cerevisiae var boulardii* et de la souche *Saccharomyces cerevisiae* inactivée, sèche induisait l'inhibition de la croissance de la plupart des pathogènes dans le modèle multi-espèces utilisé contrairement à la souche *Saccharomyces cerevisiae var boulardii* et à la levure *Saccharomyces cerevisiae* inactivée prises individuellement qui ont un spectre d'inhibition de la croissance des pathogènes oraux moindre.

### Effets Anti-Inflammatoires de Saccharomyces cerevisiae var. boulardii I-3799

Le but de l'étude présentée ci-dessous est d'évaluer les potentiels effets anti-inflammatoires de la levure *Saccharomyces cerevisiae* var. *boulardii* CNCM I-3799 sur les paramètres inflammatoires de monocytes primaires humains et de fibroblastes gingivaux.

### A. Evaluation du Potentiel Anti-Inflammatoire sur des Monocytes Primaires Humains

***Stimulation et Détermination des Paramètres Inflammatoires dans des Monocytes Primaires Humains.*** Des monocytes primaires humains ont été isolés et enrichis à partir de couches leucocytaires de donneurs de sang humains sains. Les cellules ont été ensemencées dans des plaques à 24 puits (environ 500 000 cellules/ml dans 1 ml) pour les expériences d'ELISA. Les cellules ont été incubées avec du LPS (10 ng/ml) pendant 24 heures. Les échantillons de levures (5 doses comprises entre 4.3^{E}6 CFU et 4.3^{E}8 CFU/ml, soit: 0,1 mg/ml; 1 mg/ml; 2,5 mg/ml; 5 mg/ml; 10 mg/ml) ont été ajoutés 30 minutes avant le traitement au LPS directement dans la culture cellulaire. La déxaméthasone a été utilisée comme contrôle positif (2 doses, 1 µM et 10 µM). Après 24 heures, les surnageants ont été retirés, centrifugés et les concentrations en MCP-1, IL-8, IL-6, IL-10, isoprostane et PGE2 ont été évaluées. Les concentrations dans les EIA (PGE2 et isoprostane, de Cayman) ou les ELISAs (MCP-1, IL-6 et IL-8, de eBioscience) ont été évalués selon les protocoles du fabricant. Chaque dose a été étudiée 6 fois à partir de couches leucocytaires issues de 3 donneurs différents (n=2 par couche leucocytaire, n=6 au total).

***Analyse Statistique.*** ANOVA unidirectionnelle avec tests de comparaison multiples de Dunnett à un niveau de signification de 0,5.

***Résultats.*** Les résultats obtenus sont présentés sur la Figure 4. Ces résultats montrent que la souche de *Saccharomyces cerevisiae var boulardii* CNCM I-3799 a un effet anti-inflammatoire dose-dépendent dans les monocytes primaires humains. Plus précisément, *S. boulardii* CNCM I-3799 est capable d'inhiber à une dose comprise entre 2.5 mg/ml (1^{E}8 CFU/ml) et 10 mg/ml (4.3^{E}8 CFU/ml) la sécrétion d'IL-6, IL-10, MCP-1, PGE-2 et d'isoprostane alors que la cytokine IL-8 est inhibée à une dose minimale de 10 mg/ml (4.3^{E}8 CFU/ml). A faible dose (de 0.1 à 1 mg/ml soit de 4.3^{E}6 à 4.3^{E}7 CFU/ml), *S. boulardii* CNCM I-3799 est capable de stimuler la sécrétion de la cytokine anti-inflammatoire Il-10 suggérant un effet « immune-training ». A plus fortes doses, l'inhibition des cytokines pro-inflammatoires et des marqueurs PGE-2 / isoprostane indiquent un effet anti-inflammatoire et une inhibition de la voie des prostaglandines impliquée dans l'apparition de la douleur et de la destruction osseuse.

### B. Evaluation du Potentiel Anti-Inflammatoire sur des Fibroblastes Gingivaux Primaires Humains

***Mesure de IL-8, IL-6, Isoprostane et PGE2 dans des Fibroblastes Gingivaux Humains Primaires.*** Des cultures de fibroblastes gingivaux primaires humains fournies par Provitro (Berlin, Allemagne) ont été maintenues suivant le protocole du fournisseur. Avant la stimulation, les cellules ont été ensemencées dans des plaques à 24 puits pour les expériences d'ELISA. Les cellules ont été incubées sans (contrôle non stimulé) ou avec IL-1β (10 U/ml) pendant 24 heures. Les échantillons de levures ont été ajoutés 30 minutes avant le traitement par IL-1β en 5 doses (0,1 mg/ml; 1 mg/ml; 2,5 mg/ml; 5 mg/ml; 10 mg/ml) (n=3). La déxaméthasone a été utilisée comme contrôle positif (1 dose, 1 µM). Après 24 heures, les surnageants ont été prélevés, centrifugés et les concentrations en IL-6, IL-8, isoprostane et PGE2 ont été évaluées par EIA (PGE2 et isoprostane, de Cayman) ou par ELISA (IL-6 et IL-8, de eBioscience) en suivant le protocole du fabricant. Chaque dose a été étudiée au moins 3 fois.

***Résultats.*** Les résultats obtenus sont présentés sur la Figure 5. Ces résultats montrent que la souche de *Saccharomyces cerevisiae var boulardii* CNCM I-3799 a un effet anti-inflammatoire dose-dépendent dans les fibroblastes gingivaux primaires humains. Plus précisément, *S. boulardii* CNCM I-3799 est capable d'inhiber à une dose comprise entre 1 mg/ml (4.3^{E}7 CFU/ml) et 10 mg/ml (4.3^{E}8 CFU/ml) la sécrétion d'IL-6, IL-8, PGE-2 et d'isoprostane. L'inhibition des cytokines pro-inflammatoires et des marqueurs PGE-2 / isoprostane indiquent un effet anti-inflammatoire et une inhibition de la voie des prostaglandines impliquée dans l'apparition de la douleur et de la destruction osseuse.

## Revendications

1. Souche de levure *Saccharomyces cerevisiae var boulardii* déposée, le 21 août 2007, auprès de la CNCM sous le numéro I-3799,
ou
combinaison de ladite souche de levure *Saccharomyces cerevisiae var boulardii* avec la forme sèche inactivée d'une souche de levure *Saccharomyces cerevisiae,* pour utilisation dans la prévention et/ou le traitement d'une maladie infectieuse de la cavité buccale chez un sujet, **caractérisée en ce que** la maladie infectieuse est une carie dentaire, une gingivite ou une parodontite.

2. Souche de levure *Saccharomyces cerevisiae var boulardii* I-3799 ou combinaison d'une souche de levure *Saccharomyces cerevisiae var boulardii* avec la forme sèche inactivée d'une souche de levure *Saccharomyces cerevisiae,* pour utilisation selon la revendication 1, **caractérisée en ce que** la levure *Saccharomyces cerevisiae var boulardii* I-3799 ou la souche de levure *Saccharomyces cerevisiae var boulardii* de la combinaison se trouve sous forme sèche vivante.

3. Souche de levure *Saccharomyces cerevisiae var boulardii* I-3799 ou combinaison d'une souche de levure *Saccharomyces cerevisiae var boulardii* avec la forme sèche inactivée d'une souche de levure *Saccharomyces cerevisiae* pour utilisation selon la revendication 1 ou la revendication 2, **caractérisée en ce que** la souche de levure *Saccharomyces cerevisiae var boulardii* de la combinaison est la souche déposée, le 21 août 2007, auprès de la CNCM sous le numéro I-3799.

4. Souche de levure *Saccharomyces cerevisiae var boulardii* I-3799 ou combinaison d'une souche de levure *Saccharomyces cerevisiae var boulardii* avec la forme sèche inactivée d'une souche de levure *Saccharomyces cerevisiae* pour utilisation selon l'une quelconque des revendications 1 à 3, **caractérisée en ce que** la souche de levure *Saccharomyces cerevisiae* est la souche déposée, le 17 octobre 2007 auprès de la CNCM sous le numéro I-3856.

5. Souche de levure *Saccharomyces cerevisiae var boulardii* I-3799 ou combinaison d'une souche de levure *Saccharomyces cerevisiae var boulardii* avec la forme sèche inactivée d'une souche de levure *Saccharomyces cerevisiae* pour utilisation selon l'une quelconque des revendications 1 à 4, **caractérisée en ce que** la souche de levure *Saccharomyces cerevisiae var boulardii* I-3799 ou la combinaison est comprise dans un complément alimentaire.

6. Souche de levure *Saccharomyces cerevisiae var boulardii* I-3799 ou combinaison d'une souche de levure *Saccharomyces cerevisiae var boulardii* avec la forme sèche inactivée d'une souche de levure *Saccharomyces cerevisiae* pour utilisation selon la revendication 5, **caractérisée en ce que** le complément alimentaire est sous forme d'une pastille à sucer, d'un bonbon, d'un chewing gum, d'une poudre orodispersible ou à diluer dans l'eau sous forme de stick ou de sachet, d'un comprimé à sucer ou à mâcher, d'une gomme à mâcher, d'une gélule, d'un comprimé, de gouttes, d'une fiole avec bouchon doseur.

7. Souche de levure *Saccharomyces cerevisiae var boulardii* I-3799 ou combinaison d'une souche de levure *Saccharomyces cerevisiae var boulardii* avec la forme sèche inactivée d'une souche de levure *Saccharomyces cerevisiae* pour utilisation selon l'une quelconque des revendications 1 à 4, **caractérisée en ce que** la souche de levure *Saccharomyces cerevisiae var boulardii* I-3799 ou la combinaison est comprise dans une composition para-pharmaceutique ou cosmétique.

8. Souche de levure *Saccharomyces cerevisiae var boulardii* I-3799 ou combinaison d'une souche de levure *Saccharomyces cerevisiae var boulardii* avec la forme sèche inactivée d'une souche de levure *Saccharomyces cerevisiae* pour utilisation selon la revendication 7, **caractérisée en ce que** la composition para-pharmaceutique ou cosmétique est sous forme d'un dentifrice, d'un bain de bouche, d'un spray oral, d'une crème ou d'un gel oral, d'une feuille orodispersible, d'une poudre destinée à être saupoudrée directement dans la cavité buccale, d'une poudre orodispersible ou à diluer dans l'eau sous forme de stick ou de sachet, d'une fiole avec bouchon doseur.

9. Souche de levure *Saccharomyces cerevisiae var boulardii* I-3799 ou combinaison d'une souche de levure *Saccharomyces cerevisiae var boulardii* avec la forme sèche inactivée d'une souche de levure *Saccharomyces cerevisiae* pour utilisation selon l'une quelconque des revendications 1 à 4, **caractérisée en ce que** la souche de levure *Saccharomyces cerevisiae var boulardii* I-3799 ou la combinaison est comprise dans une composition pharmaceutique, la composition pharmaceutique comprenant une quantité efficace de la souche de levure *Saccharomyces cerevisiae var boulardii* I-3799, ou de la combinaison, définie dans l'une quelconque des revendications 1 à 4, et au moins un excipient physiologiquement acceptable.

10. Souche de levure *Saccharomyces cerevisiae var boulardii* I-3799 ou combinaison d'une souche de levure *Saccharomyces cerevisiae var boulardii* avec la forme sèche inactivée d'une souche de levure *Saccharomyces cerevisiae* pour utilisation selon la revendication 9, **caractérisée en ce que** la composition pharmaceutique est destinée à une administration topique ou à une administration par voie orale.

11. Souche de levure *Saccharomyces cerevisiae var boulardii* I-3799 ou combinaison d'une souche de levure *Saccharomyces cerevisiae var boulardii* avec la forme sèche inactivée d'une souche de levure *Saccharomyces cerevisiae* pour son utilisation selon la revendication 9 ou la revendication 10, **caractérisée en ce que** la composition pharmaceutique comprend en outre au moins un principe actif pharmaceutique supplémentaire ayant une activité apaisante, anti-irritante, analgésique, antalgique, anti-inflammatoire, cicatrisante, antibiotique, antipyrétique, ou antifongique.

12. Souche de levure *Saccharomyces cerevisiae var boulardii* I-3799 ou combinaison d'une souche de levure *Saccharomyces cerevisiae var boulardii* avec la forme sèche inactivée d'une souche de levure *Saccharomyces cerevisiae* pour utilisation selon l'une quelconque des revendications 1 à 4, **caractérisée en ce que** la souche de levure *Saccharomyces cerevisiae var boulardii* I-3799 ou la combinaison est comprise dans un appareil médical dentaire.

13. Souche de levure *Saccharomyces cerevisiae var boulardii* I-3799 ou combinaison d'une souche de levure *Saccharomyces cerevisiae var boulardii* avec la forme sèche inactivée d'une souche de levure *Saccharomyces cerevisiae* pour utilisation selon la revendication 12, **caractérisé en ce que** l'appareil médical dentaire est un implant dentaire, une couronne dentaire, un bridge dentaire, un onlay dentaire, ou une prothèse dentaire.

14. Souche de levure *Saccharomyces cerevisiae var boulardii* I-3799 ou combinaison d'une souche de levure *Saccharomyces cerevisiae var boulardii* avec la forme sèche inactivée d'une souche de levure *Saccharomyces cerevisiae* pour utilisation selon l'une quelconque des revendications 1 à 13, **caractérisée en ce que** la maladie infectieuse de la cavité buccale est induite par des bactéries parodontopathogènes et/ou des bactéries cariopathogènes.

15. Souche de levure *Saccharomyces cerevisiae var boulardii* I-3799 ou combinaison d'une souche de levure *Saccharomyces cerevisiae var boulardii* avec la forme sèche inactivée d'une souche de levure *Saccharomyces cerevisiae* pour utilisation selon la revendication 14, **caractérisée en ce que** les bactéries parodontopathogènes sont choisies parmi *Aggregatibacter actinomycetemcomitans, Fusobacterium nucleatum*)*, Porphyromonas gingivalis, Prevotella intermedia,* et/ou la bactérie cariopathogène est *Streptococcus mutans.*

16. Souche de levure *Saccharomyces cerevisiae var boulardii* I-3799 ou combinaison d'une souche de levure *Saccharomyces cerevisiae var boulardii* avec la forme sèche inactivée d'une souche de levure *Saccharomyces cerevisiae* pour utilisation selon l'une quelconque des revendications 1 à 15, **caractérisée en ce que** la maladie infectieuse de la cavité buccale est un effet secondaire d'un traitement médical, ou **en ce que** la maladie infectieuse de la cavité buccale est présente ou susceptible de se développer ou de récidiver chez un patient souffrant d'une immunovulnérabilité, ou **en ce que** le sujet est une femme enceinte, une personne âgée, un enfant ou un patient présentant un phénotype hyper-inflammatoire.

## Patentansprüche

1. Hefestamm *Saccharomyces cerevisiae var boulardii,* der am 21. August 2007 bei der CNCM unter der Nummer I-3799 hinterlegt wurde, oder Kombination des Hefestamms *Saccharomyces cerevisiae var boulardii* mit der inaktivierten Trockenform eines Hefestamms *Saccharomyces cerevisiae,* zur Verwendung bei der Prävention und/oder Behandlung einer Infektionskrankheit der Mundhöhle bei einem Patienten, **dadurch gekennzeichnet, dass** die Infektionskrankheit Karies, Gingivitis oder Parodontitis ist.

2. Hefestamm *Saccharomyces cerevisiae var boulardii* I-3799 oder Kombination eines Hefestamms *Saccharomyces cerevisiae var boulardii* mit der inaktivierten Trockenform eines Hefestamms *Saccharomyces cerevisiae,* zur Verwendung nach Anspruch 1, **dadurch gekennzeichnet, dass** die Hefe *Saccharomyces cerevisiae var boulardii* I-3799 oder der Hefestamm *Saccharomyces cerevisiae var boulardii* der Kombination in lebender Trockenform vorliegt.

3. Hefestamm *Saccharomyces cerevisiae var boulardii* I-3799 oder Kombination eines Hefestamms *Saccharomyces cerevisiae var boulardii* mit der inaktivierten Trockenform eines Hefestamms *Saccharomyces cerevisiae* zur Verwendung nach Anspruch 1 oder Anspruch 2, **dadurch gekennzeichnet, dass** der Hefestamm *Saccharomyces cerevisiae var boulardii* der Kombination der Stamm ist, der am 21. August 2007 bei der CNCM unter der Nummer I-3799 hinterlegt wurde.

4. Hefestamm *Saccharomyces cerevisiae var boulardii* I-3799 oder Kombination eines Hefestamms *Saccharomyces cerevisiae var boulardii* mit der inaktivierten Trockenform eines Hefestamms *Saccharomyces cerevisiae* zur Verwendung nach eines der Ansprüche 1 bis 3, **dadurch gekennzeichnet, dass** der Hefestamm *Saccharomyces cerevisiae* der Stamm ist, der am 17. Oktober 2007 bei der CNCM unter der Nummer 1-3856 hinterlegt wurde.

5. Hefestamm *Saccharomyces cerevisiae var boulardii* I-3799 oder Kombination eines Hefestamms *Saccharomyces cerevisiae var boulardii* mit der inaktivierten Trockenform eines Hefestamms *Saccharomyces cerevisiae* zur Verwendung nach einem der Ansprüche 1 bis 4, **dadurch gekennzeichnet, dass** der Hefestamm *Saccharomyces cerevisiae var boulardii* I-3799 oder die Kombination in einem Nahrungsergänzungsmittel enthalten ist.

6. Hefestamm *Saccharomyces cerevisiae var boulardii* I-3799 oder Kombination eines Hefestamms *Saccharomyces cerevisiae var boulardii* mit der inaktivierten Trockenform eines Hefestamms *Saccharomyces cerevisiae* zur Verwendung nach Anspruch 5, **dadurch gekennzeichnet, dass** das Nahrungsergänzungsmittel in Form einer Lutschpastille, eines Bonbons, einem Kaugummi, einem in Wasser löslichen Pulver in Form eines Sticks oder Beutels, einer Lutsch- oder Kautablette, einem Kaugummi, einer Kapsel, einer Tablette, Tropfen, einer Ampulle mit Dosierkappe vorliegt.

7. Hefestamm *Saccharomyces cerevisiae var boulardii* I-3799 oder Kombination eines Hefestamms *Saccharomyces cerevisiae var boulardii* mit der inaktivierten Trockenform eines Hefestamms *Saccharomyces cerevisiae* zur Verwendung nach einem der Ansprüche 1 bis 4, **dadurch gekennzeichnet, dass** der Hefestamm *Saccharomyces cerevisiae var boulardii* I-3799 oder die Kombination in einer parapharmazeutischen oder kosmetischen Zusammensetzung enthalten ist.

8. Hefestamm *Saccharomyces cerevisiae var boulardii* I-3799 oder Kombination eines Hefestamms *Saccharomyces cerevisiae var boulardii* mit der inaktivierten Trockenform eines Hefestamms *Saccharomyces cerevisiae* zur Verwendung nach Anspruch 7, **dadurch gekennzeichnet, dass** die parapharmazeutische oder kosmetische Zusammensetzung in Form von Zahnpasta, Mundwasser, Mundspray, Mundcreme oder -gel, Schmelz- oder Kautablette, Pulver zum direkten Einstreuen in die Mundhöhle, Pulver zum Auflösen in Wasser in Form eines Sticks oder Beutels, einer Ampulle mit Dosierkappe vorliegt.

9. Hefestamm *Saccharomyces cerevisiae var boulardii* I-3799 oder Kombination eines Hefestamms *Saccharomyces cerevisiae var boulardii* mit der inaktivierten Trockenform eines Hefestamms *Saccharomyces cerevisiae* zur Verwendung nach einem der Ansprüche 1 bis 4, **dadurch gekennzeichnet, dass** der Hefestamm *Saccharomyces cerevisiae var boulardii* I-3799 oder die Kombination in einer pharmazeutischen Zusammensetzung enthalten ist, wobei die pharmazeutische Zusammensetzung eine wirksame Menge des Hefestamms *Saccharomyces cerevisiae var boulardii* I-3799 oder der Kombination, wie in einem der Ansprüche 1 bis 4 definiert, und wenigstens einen physiologisch akzeptablen Träger umfasst.

10. Hefestamm *Saccharomyces cerevisiae var boulardii* I-3799 oder Kombination eines Hefestamms *Saccharomyces cerevisiae var boulardii* mit der inaktivierten Trockenform eines Hefestamms *Saccharomyces cerevisiae* zur Verwendung nach Anspruch 9, **dadurch gekennzeichnet, dass** die pharmazeutische Zusammensetzung zur topischen oder oralen Verabreichung bestimmt ist.

11. Hefestamm *Saccharomyces cerevisiae var boulardii* I-3799 oder Kombination eines Hefestamms *Saccharomyces cerevisiae var boulardii* mit der inaktivierten Trockenform eines Hefestamms *Saccharomyces cerevisiae* zur Verwendung nach Anspruch 9 oder Anspruch 10, **dadurch gekennzeichnet, dass** die pharmazeutische Zusammensetzung ferner wenigstens einen weiteren pharmazeutischen Wirkstoff mit beruhigender, reizlindernder, analgetischer, entzündungshemmender, wundheilender, antibiotischer, fiebersenkender oder antimykotischer Wirkung umfasst.

12. Hefestamm *Saccharomyces cerevisiae var boulardii* I-3799 oder Kombination eines Hefestamms *Saccharomyces cerevisiae var boulardii* mit der inaktivierten Trockenform eines Hefestamms *Saccharomyces cerevisiae* zur Verwendung nach einem der Ansprüche 1 bis 4, **dadurch gekennzeichnet, dass** der Hefestamm *Saccharomyces cerevisiae var boulardii* I-3799 oder die Kombination in einer zahnmedizinischen Vorrichtung enthalten ist.

13. Hefestamm *Saccharomyces cerevisiae var boulardii* I-3799 oder Kombination eines Hefestamms *Saccharomyces cerevisiae var boulardii* mit der inaktivierten Trockenform eines Hefestamms *Saccharomyces cerevisiae* zur Verwendung nach Anspruch 12, **dadurch gekennzeichnet, dass** die zahnmedizinische Vorrichtung ein Zahnimplantat, eine Zahnkrone, eine Zahnbrücke, ein Onlay oder eine Zahnprothese ist.

14. Hefestamm *Saccharomyces cerevisiae var boulardii* I-3799 oder Kombination eines Hefestamms *Saccharomyces cerevisiae var boulardii* mit der inaktivierten Trockenform eines Hefestamms *Saccharomyces cerevisiae* zur Verwendung nach einem der Ansprüche 1 bis 13, **dadurch gekennzeichnet, dass** die Infektionskrankheit der Mundhöhle durch parodontopathogene Bakterien und/oder kariopathogene Bakterien induziert wird.

15. Hefestamm *Saccharomyces cerevisiae var boulardii* I-3799 oder Kombination eines Hefestamms *Saccharomyces cerevisiae var boulardii* mit der inaktivierten Trockenform eines eines Hefestamms *Saccharomyces cerevisiae* zur Verwendung nach Anspruch 14, **dadurch gekennzeichnet, dass** die parodontopathogenen Bakterien gewählt sind aus *Aggregatibacter actinomycetemcomitans, Fusobacterium nucleatum), Porphyromonas gingivalis, Prevotella intermedia,* und/oder das kariopathogene Bakterium *Streptococcus mutans ist.*

16. Hefestamm *Saccharomyces cerevisiae var boulardii* I-3799 oder Kombination eines Hefestamms *Saccharomyces cerevisiae var boulardii* mit der inaktivierten Trockenform eines Hefestamms *Saccharomyces cerevisiae* zur Verwendung nach einem der Ansprüche 1 bis 15, **dadurch gekennzeichnet, dass** die Infektionskrankheit der Mundhöhle eine Nebenwirkung einer medizinischen Behandlung ist oder dass die Infektionskrankheit der Mundhöhle bei einem Patienten mit einer Immunschwäche vorliegt oder sich entwickeln oder rezidivieren kann, oder dass es sich bei der Person um eine schwangere Frau, eine ältere Person, ein Kind oder einen Patienten mit einem hyperinflammatorischen Phänotyp handelt.

## Claims

1. Yeast strain of *Saccharomyces cerevisiae var boulardii* deposited on 21 August 2007 with the CNCM under number I-3799,
or
combination of said yeast strain of *Saccharomyces cerevisiae var boulardii* with the inactivated dry form of a yeast strain of *Saccharomyces cerevisiae,*
for use in the prevention and/or treatment of an infectious disease of the oral cavity in a subject, **characterised in that** the infectious disease is tooth decay, gingivitis or periodontitis.

2. Yeast strain of *Saccharomyces cerevisiae var boulardii* I-3799 or combination of a yeast strain of *Saccharomyces cerevisiae var boulardii* with the inactivated dry form of a yeast strain of *Saccharomyces cerevisiae,* for use according to claim 1, **characterised in that** the *Saccharomyces cerevisiae var boulardii* yeast I-3799 or the yeast strain of *Saccharomyces* cerevisiae var boulardii of the combination is in the live dry form.

3. Yeast strain of *Saccharomyces cerevisiae var boulardii* I-3799 or combination of a yeast strain of *Saccharomyces cerevisiae var boulardii* with the inactivated dry form of a yeast strain of *Saccharomyces cerevisiae* for use according to claim 1 or claim 2, **characterised in that** the yeast strain of *Saccharomyces cerevisiae var boulardii* of the combination is the strain deposited on 21 August 2007 with the CNCM under number I-3799.

4. Yeast strain of *Saccharomyces cerevisiae var boulardii* I-3799 or combination of a yeast strain of *Saccharomyces cerevisiae var boulardii* with the inactivated dry form of a yeast strain of *Saccharomyces cerevisiae* for use according to any one of claims 1 to 3, **characterised in that** the yeast strain of *Saccharomyces cerevisiae* is the strain deposited on 17 October 2007 with the CNCM under number I-3856.

5. Yeast strain of *Saccharomyces cerevisiae var boulardii* I-3799 or combination of a yeast strain of *Saccharomyces cerevisiae var boulardii* with the inactivated dry form of a yeast strain of *Saccharomyces cerevisiae* for use according to any one of claims 1 to 4, **characterised in that** the yeast strain of *Saccharomyces cerevisiae var boulardii* I-3799 or the combination is comprised in a food supplement.

6. Yeast strain of *Saccharomyces cerevisiae var boulardii* I-3799 or combination of a yeast strain of *Saccharomyces cerevisiae var boulardii* with the inactivated dry form of a yeast strain of *Saccharomyces cerevisiae* for use according to claim 5, **characterised in that** the food supplement is in the form of a pastille, a sweet, a chewing gum, an orodispersible powder or a powder to be diluted in water in the form of a stick or a sachet, a lozenge or chewable tablet, a chewing gum, a capsule, a tablet, drops, a vial with a measuring cap.

7. Yeast strain of *Saccharomyces cerevisiae var boulardii* I-3799 or combination of a yeast strain of *Saccharomyces cerevisiae var boulardii* with the inactivated dry form of a yeast strain of *Saccharomyces cerevisiae* for use according to any one of claims 1 to 4, **characterised in that** the yeast strain of *Saccharomyces cerevisiae var boulardii* I-3799 or the combination is comprised in a parapharmaceutical or cosmetic composition.

8. Yeast strain of *Saccharomyces cerevisiae var boulardii* I-3799 or combination of a yeast strain of *Saccharomyces cerevisiae var boulardii* with the inactivated dry form of a yeast strain of *Saccharomyces cerevisiae* for use according to claim 7, **characterised in that** the parapharmaceutical or cosmetic composition is in the form of a toothpaste, a mouthwash, an oral spray, an oral cream or gel, an orodispersible sheet, a powder intended to be sprinkled directly into the oral cavity, an orodispersible powder or a powder to be diluted in water in the form of a stick or sachet, a vial with a measuring cap.

9. Yeast strain of *Saccharomyces cerevisiae var boulardii* I-3799 or combination of a yeast strain of *Saccharomyces cerevisiae var boulardii* with the inactivated dry form of a yeast strain of *Saccharomyces cerevisiae* for use according to any one of claims 1 to 4, **characterised in that** the yeast strain of *Saccharomyces cerevisiae var boulardii* I-3799 or the combination is comprised in a pharmaceutical composition, the pharmaceutical composition comprising an effective amount of the yeast strain of *Saccharomyces cerevisiae var boulardii* 1-3799, or the combination defined in any one of claims 1 to 4 and at least one physiologically acceptable excipient.

10. Yeast strain of *Saccharomyces cerevisiae var boulardii* I-3799 or combination of a yeast strain of *Saccharomyces cerevisiae var boulardii* with the inactivated dry form of a yeast strain of *Saccharomyces cerevisiae* for use according to claim 9, **characterised in that** the pharmaceutical composition is intended for topical administration or for oral administration.

11. Yeast strain of *Saccharomyces cerevisiae var boulardii* I-3799 or combination of a yeast strain of *Saccharomyces cerevisiae var boulardii* with the inactivated dry form of a yeast strain of *Saccharomyces cerevisiae* for use according to claim 9 or claim 10, **characterised in that** the pharmaceutical composition further comprises at least one additional pharmaceutical active ingredient having a soothing, anti-irritant, analgesic, anti-pain, anti-inflammatory, healing, antibiotic, antipyretic or antifungal effect.

12. Yeast strain of *Saccharomyces cerevisiae var boulardii* I-3799 or combination of a yeast strain of *Saccharomyces cerevisiae var boulardii* with the inactivated dry form of a yeast strain of *Saccharomyces cerevisiae* for use according to any one of claims 1 to 4, **characterised in that** the yeast strain of *Saccharomyces cerevisiae var boulardii* I-3799 or the combination is comprised in a dental medical device.

13. Yeast strain of *Saccharomyces cerevisiae var boulardii* I-3799 or combination of a yeast strain of *Saccharomyces cerevisiae var boulardii* with the inactivated dry form of a yeast strain of *Saccharomyces cerevisiae* for use according to claim 12, **characterised in that** the dental medical device is a dental implant, a dental crown, a dental bridge, a dental onlay, or a dental prosthesis.

14. Yeast strain of *Saccharomyces cerevisiae var boulardii* I-3799 or combination of a yeast strain of *Saccharomyces cerevisiae var boulardii* with the inactivated dry form of a yeast strain of *Saccharomyces cerevisiae* for use according to any one of claims 1 to 13, **characterised in that** the infectious disease of the oral cavity is caused by periodontopathogenic and/or cariopathogenic bacteria.

15. Yeast strain of *Saccharomyces cerevisiae var boulardii* I-3799 or combination of a yeast strain of *Saccharomyces cerevisiae var boulardii* with the inactivated dry form of a yeast strain of *Saccharomyces cerevisiae* for use according to claim 14, **characterised in that** the periodontopathogenic bacteria are selected from *Aggregatibacter actinomycetemcomitans, Fusobacterium nucleatum, Porphyromonas gingivalis, Prevotella intermedia,* and/or the cariopathogenic bacteria is *Streptococcus mutans.*

16. Yeast strain of *Saccharomyces cerevisiae var boulardii* yeast strain I-3799 or combination of a yeast strain of *Saccharomyces cerevisiae var boulardii* with the inactivated dry form of a yeast strain of *Saccharomyces cerevisiae* for use according to any one of claims 1 to 15, **characterised in that** the infectious disease of the oral cavity is a side effect of a medical treatment, or **in that** the infectious disease of the oral cavity is present or likely to develop or recur in a patient suffering from immunovulnerability, or **in that** the subject is a pregnant woman, an elderly person, a child, or a patient with a hyperinflammatory phenotype.
